# EUROPEAN PATENT SPECIFICATION

(11) **EP 1 960 536 B1**
(45) Date of publication and mention of the grant of the patent: **26.06.2013**
(21) Application number: 06817468.9
(22) Date of filing: 29.11.2006
(51) Int. Cl.: C12Q 1/68

(54) **DETECTION OF FUNGI**
NACHWEIS VON PILZEN
DETECTION DE CHAMPIGNONS

(30) Priority: 30.11.2005 AT 19342005
(43) Date of publication of application: 27.08.2008
(73) Proprietor: St. Anna Kinderkrebsforschung, 1090 Vienna (AT); Austria Wirtschaftsservice Gesellschaft mit beschränkter Haftung, 1030 Vienna (AT)
(72) Inventor: LION, Thomas, A-1140 Vienna (AT); BASKOVA, Lenka, 53835 Zajecice (CZ)
(74) Representative: Sonn & Partner Patentanwälte
(86) International application number: PCT/AT2006/000491
(87) International publication number: WO 2007/062442

(56) References cited:
- US-A1- 5 958 693
- US-A1- 2005 227 236
- DATABASE WPI Week 200454 Derwent Publications Ltd., London, GB; AN 2004-556368 XP002423120 -& JP 2004 201641 A (MITSUBISHI YUKA BCL KK) 22 July 2004 (2004-07-22)
- HINRIKSON H P ET AL: "MOLECULAR METHODS FOR THE IDENTIFICATION OF ASPERGILLUS SPECIES" MEDICAL MYCOLOGY, OXFORD, GB, vol. 43, no. SUPPL 1, May 2005 (2005-05), pages S129-S137, XP009068121 ISSN: 1369-3786
- EVERTSSON U ET AL: "DETECTION AND IDENTIFICATION OF FUNGI IN BLOOD USING BROAD-RANGE 28S RDNA PCR AMPLIFICATION AND SPECIES-SPECIFIC HYBRIDISATION" APMIS, COPENHAGEN, DK, vol. 108, no. 5, 2000, pages 385-392, XP008010084 ISSN: 0903-4641 cited in the application

### SEQUENCE LISTING

<110> St. Anna Kinderkrebsforschung
   Austria Wirtschaftssrevice GmbH
<120> Detection of fungi
<130> R 48774
<140>
   <141>
<150> AT A 1934/2005 <151> 2005-11-30
<160> 35
<170> PatentIn version 3.3
<210> 1
   <211> 100
   <212> DNA
   <213> Artificial
<220>
   <223> Sequence Alignment
<400> 1
   ctctaaatgg gtggtaaatt tcatctaaag ctaaatacyg gccggagacc gatagcgcac 60
   aagtagagtg atcgaaagat gaaaagcact ttgaaaagag 100
<210> 2
   <211> 100
   <212> DNA
   <213> Artificial
<220>
   <223> Sequence Alignment
<400> 2
   ctchaagygg gtggtaaayt ccatctaarg ctaaataywg gygagagacc gatagcgaac 60
   aagtacwgtg awggaaagrt gaaaagmact ttgaaaagag 100
<210> 3
   <211> 100
   <212> DNA
   <213> Artificial
<220>
   <223> Sequence Alignment
<400> 3
   ctchaarygg gtggtaaayt ycatctaarg ctaaatayhg gysrgagacc gatagcgmac 60
   aagtaswgtg awsgaaagrt gaaaagmact ttgaaaagag 100
<210> 4
   <211> 100
   <212> DNA
   <213> Artificial
<220>
   <223> Sequence Alignment
<400> 4
   ctcwaaatgg gwggtrwayb ycwtctaaag ctaaatacyg gccrgagacc gatagcgcac 60
   aagtagagtg atcgaaagat gaaaagmact ttgaaaagag 100
<210> 5
   <211> 100
   <212> DNA
   <213> Artificial
<220>
   <223> Sequence Alignment
<400> 5
   cyyhaadykg gtggtraaty ycayctaarg ctaaataywk gcgagaracc gatagcgaac 60
   aagtachgtg adggaaagat gaaaaghact ttgraaagag 100
<210> 6
   <211> 136
   <212> DNA
   <213> Artificial
<220>
   <223> sequence Alignment
<400> 6
   agyhccttcr acgagtcgag twgtttggga atgcwgctcw aaatggrwgg tahaybycwt 60
   ctaaagctaa atayhgkycr gagaccgata gcgcacaagt agagtgatcg aaagatgaaa 120
   agmactttgr aaagag 136
<210> 7
   <211> 128
   <212> DNA
   <213> Artificial
<220>
   <223> Sequence Alignment
<400> 7
   sdahgagtcr vgttryttgg gadtgyagcb yhaadydggw ggtrrryyyc wyctaargct 60
   aaatayhkvy grraraccga tagcgaacaa gtachgtgad ggaaagrtga aaaghactyt 120
   graaagag 128
<210> 8
   <211> 26
   <212> DNA
   <213> Artificial
<220>
   <223> oligonucleotide
<400> 8
   gggtggtaaa ttccatctaa rgctaa 26
<210> 9
   <211> 26
   <212> DNA
   <213> Artificial
<220>
   <223> Oligonucleotide
<400> 9
   gggtggtaaa tttcatctaa agctaa 26
<210> 10
   <211> 26
   <212> DNA
   <213> Artificial
<220>
   <223> oligonucleotide
<400> 10
   gggtggtaaa ttycatctaa rgctaa 26
<210> 11
   <211> 23
   <212> DNA
   <213> Artificial
<220>
   <223> oligonucleotide
<400> 11
   taaagctaaa tayyggccrg aga 23
<210> 12
   <211> 22
   <212> DNA
   <213> Artificial
<220>
   <223> oligonucleotide
<400> 12
   gagtcgactw gtttgggaat gc 22
<210> 13
   <211> 26
   <212> DNA
   <213> Artificial
<220>
   <223> oligonucleotide
<400> 13
   gggtggtrar ytccwtctaa rgctaa 26
<210> 14
   <211> 26
   <212> DNA
   <213> Artificial
<220>
   <223> oligonucleotide
<400> 14
   gggwggtaaa tcycwcctaa agctaa 26
<210> 15
   <211> 22
   <212> DNA
   <213> Artificial
<220>
   <223> Oligonucleotide
<400> 15
   gtcrrgtwgt ttgggawtgc wg 22
<210> 16
   <211> 21
   <212> DNA
   <213> Artificial
<220>
   <223> Oligonucleotide
<400> 16
   cgcgttactt gggagtgtag c 21
<210> 17
   <211> 27
   <212> DNA
   <213> Artificial
<220>
   <223> Oligonucleotide
<400> 17
   aagttctttt catctttccw tcacwgt 27
<210> 18
   <211> 27
   <212> DNA
   <213> Artificial
<220>
   <223> oligonucleotide
<400> 18
   aagttctttt catctttcga tcactct 27
<210> 19
   <211> 21
   <212> DNA
   <213> Artificial
<220>
   <223> Oligonucleotide
<400> 19
   ctttycaaag tgcttttcat c 21
<210> 20
   <211> 23
   <212> DNA
   <213> Artificial
<220>
   <223> oligonucleotide
<400> 20
   ctcttttcaa agtwcttttc ayc 23
<210> 21
   <211> 21
   <212> DNA
   <213> Artificial
<220>
   <223> oligonucleotide
<400> 21
   cttttcaaag tkcttttcar c 21
<210> 22
   <211> 21
   <212> DNA
   <213> Artificial
<220>
   <223> oligonucleotide
<400> 22
   cttttcaaag tgcttttcat c 21
<210> 23
   <211> 27
   <212> DNA
   <213> Artificial
<220>
   <223> oligonucleotide
<400> 23
   aagtkctttt catctttcsw tcacwst 27
<210> 24
   <211> 25
   <212> DNA
   <213> Artificial
<220>
   <223> oligonucleotide
<400> 24
   cttgtkcgct atcggtctct sgcca 25
<210> 25
   <211> 15
   <212> DNA
   <213> Artificial
<220>
   <223> oligonucleotide
<400> 25
   ctatcggtyt ctcsc 15
<210> 26
   <211> 15
   <212> DNA
   <213> Artificial
<220>
   <223> oligonucleotide
<400> 26
   acttgtgcgc tatcg 15
<210> 27
   <211> 20
   <212> DNA
   <213> Artificial
<220>
   <223> Oligonucleotide
<400> 27
   tgagtatgcc tgccgtgtga 20
<210> 28
   <211> 27
   <212> DNA
   <213> Artificial
<220>
   <223> oligonucleotide
<400> 28
   actcatacac aactttcagc agcttac 27
<210> 29
   <211> 31
   <212> DNA
   <213> Artificial
<220>
   <223> oligonucleotide
<400> 29
   ccatgtgact ttgtcacagc ccaagatagt t 31
<210> 30
   <211> 21
   <212> DNA
   <213> Artificial
<220>
   <223> oligonucleotide
<400> 30
   gggcgaatca cagattgaat c 21
<210> 31
   <211> 19
   <212> DNA
   <213> Artificial
<220>
   <223> Oligonucleotide .
<400> 31
   gcggttccaa acgtaccaa 19
<210> 32
   <211> 28
   <212> DNA
   <213> Artificial
<220>
   <223> oligonucleotide
<400> 32
   tttttatgtg tccgccacca tctggatc 28
<210> 33
   <211> 16
   <212> DNA
   <213> Artificial
<220>
   <223> Oligonucleotide
<400> 33
   cttgtkcgct atcggt 16
<210> 34
   <211> 18
   <212> DNA
   <213> Artificial
<220>
   <223> oligonucleotide
<400> 34
   tacttgtkcg ctatcggt 18
<210> 35
   <211> 15
   <212> DNA
   <213> Artificial
<220>
   <223> oligonucleotide
<400> 35
   acttgttcgc tatcg 15

## Description

The present invention relates to a method for the simultaneous detection of fungi in a sample.

Invasive fungal infections (IFIs) continue to represent a significant problem in immunosuppressed individuals, including patients undergoing high-dose chemotherapy or hematopoietic stem cell transplantation, particularly for treatment of hematologic malignancies. Up to 20% of febrile patients with therapy-induced neutropenia may experience an invasive fungal infection, and autopsy studies suggest that IFIs may be encountered in as many as 40% of patients with hematologic neoplasia (Jandrlic et al.19995). Early initiation of appropriate antifungal therapy is crucial for reducing the high mortality rates associated with invasive mycoses (Morace et al.,1997).

With further advances in medical intervention and the increasing population of immunocompromised patients, the list of human fungal pathogens continues to grow. It has been estimated that more than 90% of all IFIs are caused by Aspergillus and Candida species (Hebart et al., 1997) (Singh, 2001) (Vincent at al., 1998). With the introduction of novel antifungal, antiviral and antineoplastic therapies, significant changes in the epidemiology of fungal infections have been noted. These changes include a decrease in the occurrence of candidiasis caused by the species C. albicans, C. tropicalis and C. parapsilosis, with an emergence of infections by azole-resistant species such as C. glabrata and C. krusei. In addition, an increase in the incidence of mold infections, particularly aspergillosis, has been observed together with an emergence of infections by non-fumigatus Aspergillus spp. (O'Brien et al., 2003). Additionally, a number of hitherto uncommon fungal species have emerged in recent years including infections caused by Cryptococcus, Trichosporon and Malassezia species (Golan,2005), hyalohyphomycetes such as Fusarium species (Boutati at al., 1997), different members of the class zygomycetes (Ribes et al., 2000) and phaeohyphomycetes, such as Pseudallescheria boydii (Husain et al., 2003).

The non-specific clinical features of IFIs make early diagnosis difficult. Conventional diagnostic tests including blood cultures (Vincent et al., 1998), serological detection of circulating fungal antigens (e.g. D-glucan or galactomannan) or immunoprecipitation have shown variable sensitivity and specificity (Ibanez-Nolla et al., 2001) (Machetti et al., 1998). Histological analysis of CT-guided biopsies are highly sensitive and specific, but are frequently associated with bleeding complications in patients with severe thrombocytopenia. The observation that early initiation of antifungal treatment significantly improves the outcome in neutropenic patients with IFI has provided the basis for empirical rather than evidence-based antifungal treatment of patients with febrile neutropenia (Denning,1998). Therefore, efforts are ongoing to develop less invasive, yet reliable, sensitive and specific diagnostic tests for IFIs to overcome the limitations of the traditional culture-based and serological fungus detection methods. These techniques should be replaced by rapid and sensitive procedures capable of detecting even non-culturable/non-viable cells or circulating free fungal DNA.

A method of choice can be polymerase chain reaction (PCR).Recently, a variety of PCR methods based on the detection of fungal DNA have been published (see Table 1).

**Table 1: Ribosomal rDNA gene targets for PCR-based fungal detection**

| *Gene target* | *Ref.* | *Fungus detected* | *Detection method* |
|---|---|---|---|
| **18S rDNA (SSU)** | | | |
| | (3) | Panfungal | Southern blot |
| | (3) | Panfungal | Southern blot |
| | (5) | Panfungal | RQ-PCR |
| | (3) | *Aspergillus* spp. | Nested PCR |
| | (3) | *Aspergillus spp.* | Nested PCR |
| | (3) | *Aspergillus* spp. | Nested PCR |
| | (10) | *Candida* spp. | RQ-PCR |

| **ITS1, 5.8S rDNA, ITS2** | | | |
|---|---|---|---|
| | (9) | Panfungal | Fragment analysis |
| | (3) | Panfungal | Southern blot |
| | (3) | Panfungal | Nested PCR |
| | (1) | Yeasts | Fragment analysis |
| | (2) | Yeasts | Fragment analysis |
| | (7) | Mold | RQ-PCR |
| | (3) | *Candida* spp. | Southern blot |
| | (3) | *Candida* spp. | Southern blot |
| | (4) | *Candida* spp., *Cryptococcus neoformans* | RQ-PCR |
| | (6) | *C.albicans, C.glabrata, C.krusei, C.parapsilosis, C.tropicalis* | RQ-PCR |
| | (3) | *C.albicans, C.glabrata, C.krusei, C.parapsilosis, C.kefyr, C.tropicalis* | RQ-PCR |

| **28S rDNA (LSU)** | | | |
|---|---|---|---|
| | (8) | Panfungal | Southern blot |
| | (11) | *A.flavus, A.fumigatus, A.niger, A.terreus, Paecilomyces variotii* | Nested PCR |
| | (3) | *C.albicans, C.glabrata, C.krusei, C.parapsilosis, C.tropicalis, Cryptococcus neoformans* | Southern blot |
| | (3) | *C.albicans, C. dubliniensis, C.glabrata, C.haemulonii, C.maltosa, C.parapsilosis, C.sojae, C. tropicalis, C.viswanathii., C.zeylanoides, Lodderomyces* elongisporus, *P.guilliermondii* | PCR |

*Abbreviations:* SSU, small subunit; LSU, large subunit; ITS, internal transcribed spacer; RQ-PCR, real-time quantitative PCR; References: (1) Chen, Y. C., et al. (2000) J.Clin.Microbiol. 38:2302-2310; (2) De Baere, T., et al. (2002) BMC.Microbiol. 2:21; (3) Chen, S.C., et al. (2002) Med Mycol. 40:333-57; (4) Hsu, M. C., et al. (2003) J.Med.Microbiol. 52:1071-1076; (5) Jordanides, N. E., et al. (2005) Bone Marrow Transplant 35:389-95; (6) Maaroufi, Y., et al. (2004) J Mol.Diagn. 6:108-114; (7) Pham, A. S., et al. (2003) Am.J.Clin.Pathol. 119:38-44; (8) Sandhu, G. S., et al. (1995) J.Clin.Microbiol. 33:2913-2919; (9) Turenne, C. Y., et al. (1999) J.Clin.Microbiol. 37:1846-1851; (10) White, P. L., et al. (2003) J.Med.Microbiol. 52:229-238; (11) Williamson, E. C., et al. (2000) Br.J.Haematol. 108:132-139.

Most of the published approaches rely on molecular techniques, such as nested PCR (Jaeger et al., 2000), restriction fragment length polymorphisms (RFLP) (Hopfer et al., 1993), hybridization of amplicons with specific probes employing Southern blotting methods with radioactive (e.g 32-Phosphorus [32-P]) or enzyme-labeled (e.g. digoxigenin) probes (Einsele et al., 1997) (van Burik et al., 1998), single-strand conformational polymorphisms (SSCP) (Rath et al., 2000) or sequencing of the amplified products (Ferrer et al., 2001). The major drawbacks rendering these approaches less adequate for routine laboratory use, include the labor-intensive and time consuming post-amplification handling, and the inherent high risk of false positive results due to contamination. The growing spectrum of clinically relevant fungal pathogens requires diagnostic approaches covering a large number of fungal species. Broad-spectrum PCR methods utilize so-called pan-fungal primer sets designed to recognize conserved regions among most pathogenic fungi, such as the ribosomal multi-copy rRNA gene cluster, which is the main region targeted.

Evertsoon U, et al. (APMIS (2000) 108:385-92) describe a method for the detection of fungal DNA in whole blood by amplification of 28S rDNA with nucleic acid primers and subsequent hybridization of the amplified 28S rDNA fragment with fungus species specific nucleic acid probes.

In the JP 2004201641 a method for the detection of fungi such as Candida albicans or Cryptococcus neoformans is described, which method comprises the amplification of DNA fragments with primer which are specific for fungal base sequence of 28S rRNA. The amplified nucleic acid fragments were identified with specific nucleic acid probes.

The WO 2001/07648 A1 relates to a method for the detection of prokaryotic or eukaryotic organisms comprising the amplification of DNA in a sample with primers binding to stretches of conserved sequences of the eukaryotic or prokaryotic DNA. The amplified DNA fragments were identified by using specific probes.

The US 5,849,492 relates to a method for the taxonomic assignment of fungi using 28S rRNA DNA sequences. This method comprises searching for a divergent segment of DNA. The divergent DNA segment is amplified by PCR using primers located within the conserved segments.The amplified DNA molecules are consequently compared.

In the US 5,919,617 a method for the identification of fungal pathogens using conserved regions of saccharopine dehydrogenase of Candida albicans is described.

In Hinrikson et al. (Medical Mycology 43(1) (2005): 129-137) a method for the identification of *Aspergillus* strains is disclosed. Therein the authors suggest to use "nested" PCR with primers capable to bind to the 28S rRNA region of *Aspergillus* strains.

US 5,958,693 relates to a method for detecting fungi in a sample using primers directed to the 28S rRNA region.

In US 2005/227236 a method for detecting and identifying specific fungus species is disclosed. In.the course of this method DNA fragments in the region of the 28S rRNA gene are amplified by PCR. The highly variable ITS regions of the 28S rRNA amplified by two primers are examined in order to identify a distinct species.

It is an object of the present invention to provide reliable, reproducible, highly specific, sensitive and robust methods and means for the detection of fungi, especially fungi of clinical relevance, in a sample. The method should enable a person skilled in the art to detect a broad range of fungi or a distinct fungal genus. Furthermore, it should be also possible to quantify the amount of fungi present in a sample. Such a feature would be very helpful for monitoring, for instance, the progress of a therapy.

These objects are achieved by a method for the simultaneous detection of at least one fungus, in particular mold and yeast, in a sample comprising the steps of:
a) subjecting a sample suspected of containing at least one fungus to a detection reaction I by contacting the sample with forward primer 5'-TAAAGCTAAATAYYGGCCRGAGA-3' (SEQ ID No. 11) and reverse primers 5'-CTTTYCAAAGTGCTTTTCATC-3' (SEQ ID No. 19) and 5'-CTCTTTTCAAAGTWCTTTTCAYC-3' (SEQ ID No. 20) and a nucleic acid probe having the sequence 5'-ACTTGTGCGCTATCG-3' (SEQ ID No. 26) and to a detection reaction II by contacting the sample with forward primers 5'-GGGTGGTRARYTCCWTCTAARGCTAA-3' (SEQ ID No. 13) and 5'-GGGWGGTAAATCYCWCCTAAAGCTAA-3' (SEQ ID No. 14) and with reverse primers 5'-CTTTYCAAAGTGCTTTT-CATC-3' (SEQ ID No. 19) and 5'-CTCTTTTCAAAGTWCTTTTCAYC-3' (SEQ ID No. 20), and a nucleic acid probe having the sequence 5'-ACTTGTTCGCTATCG-3' (SEQ ID No. 35), wherein detection reaction I and detection reaction II is a nucleic acid amplification technique, and
b) optionally determining the presence of the at least one fungus in said sample by detecting a nucleic acid amplification product.

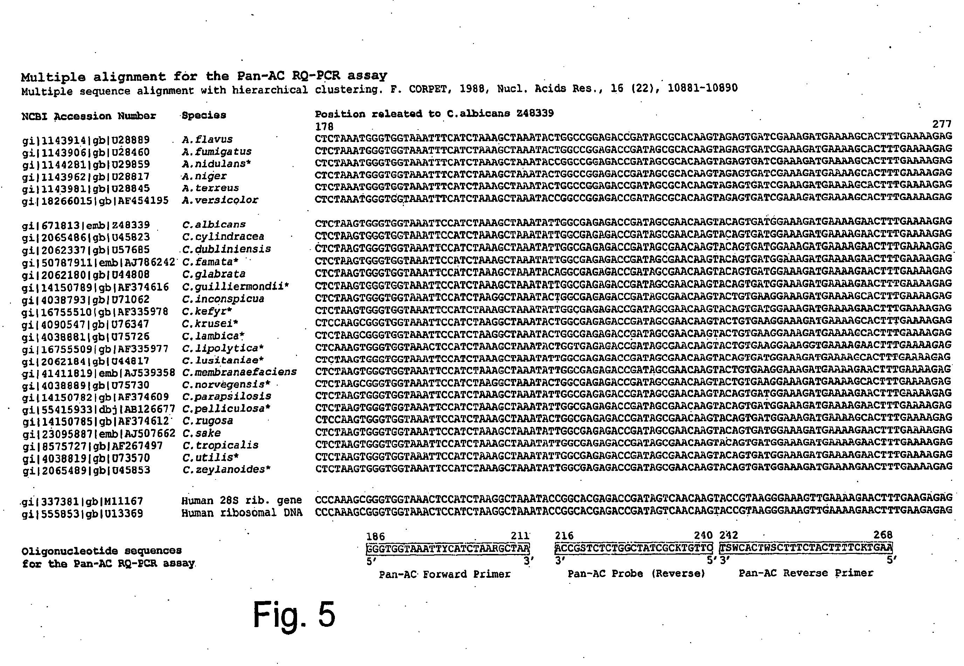

Sequence analysis, in particular sequence alignments (see Figs. 5 to 7), of a large number of 28S rRNA gene sequences of fungi (see Tables 2, 4 and 5) revealed regions which are substantially - apart from some mutations - conserved regions. The identification of said regions allowed determining primer pairs which can be employed to detect by a nucleic acid amplification technique the presence of at least one fungus in a sample.

The consensus sequence SEQ ID No. 1 is derived from an alignment of 28S rRNA of various members of the Aspergillus genus (see Table 2) and shown in Fig. 5 upper part. SEQ ID No. 2, derived from an alignment of 28S rRNA of various members of the Candida genus (see Table 2), is shown in Fig. 5 lower part. The consensus sequence of both Aspergillus and Candida genera results in SEQ ID No. 3.

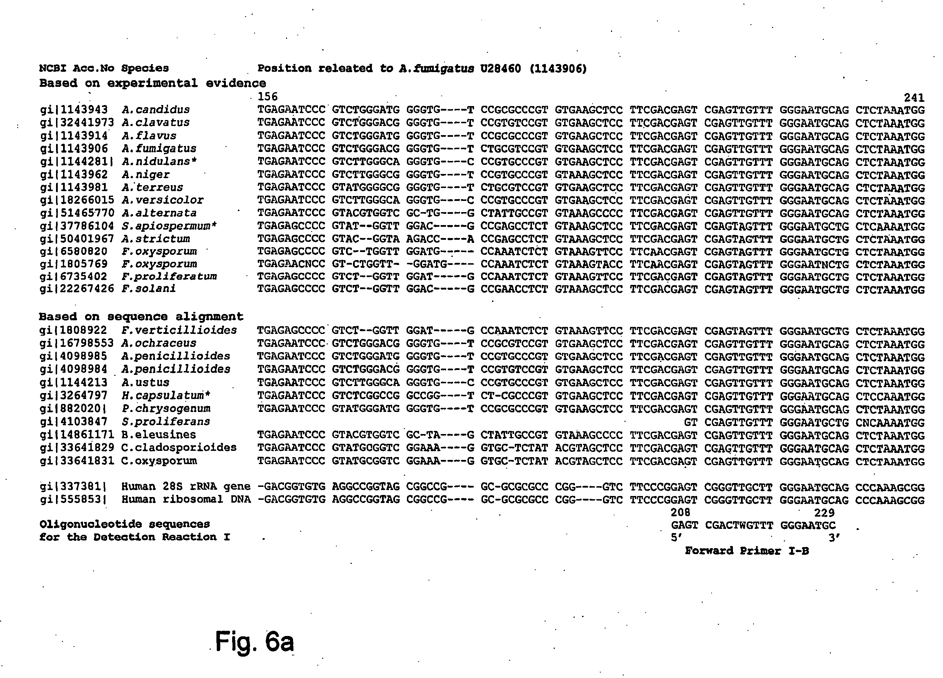

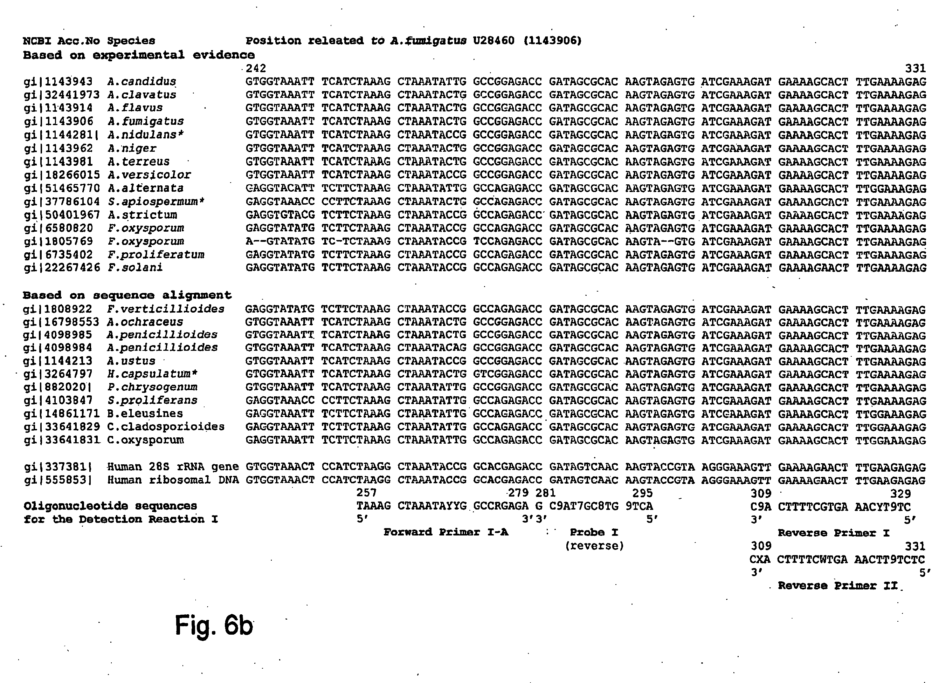

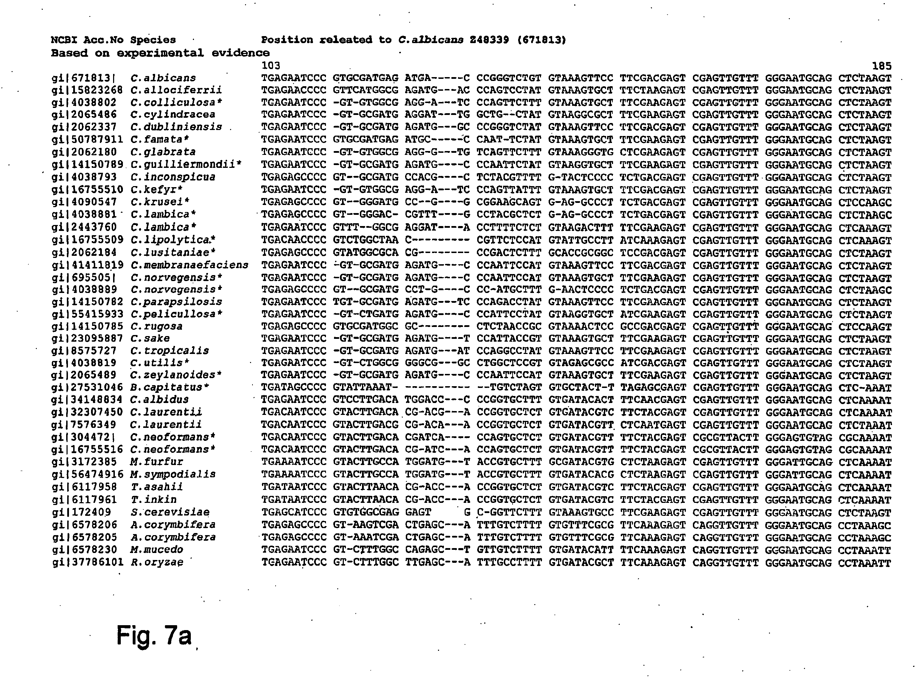

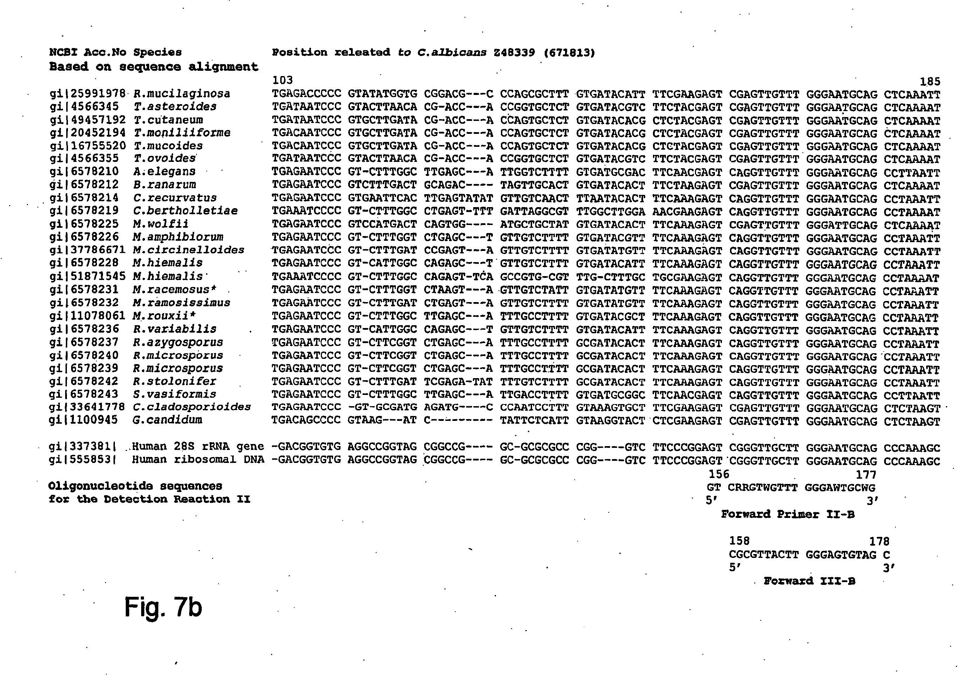

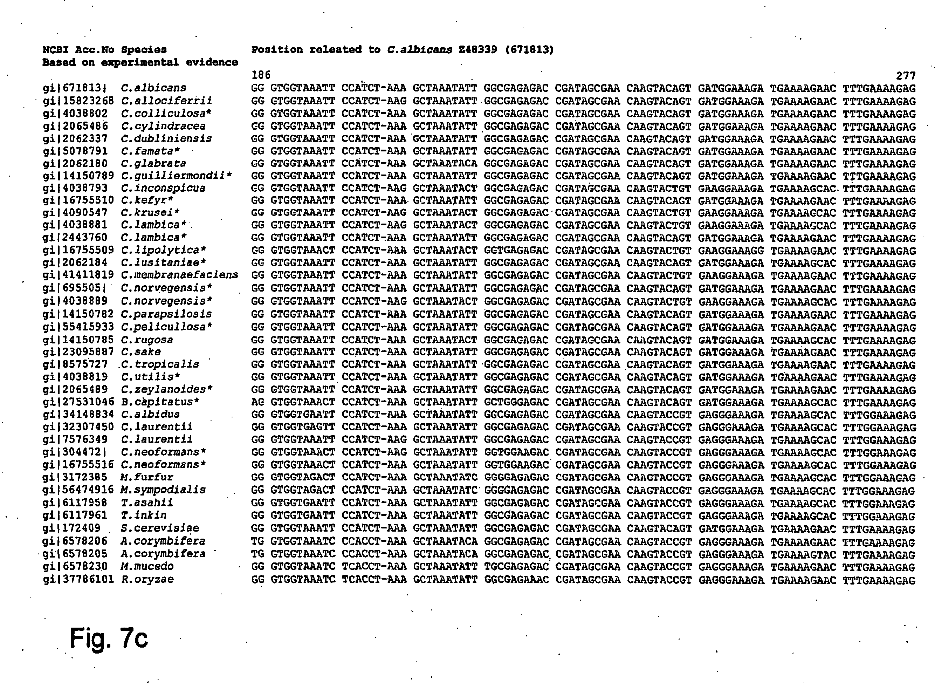

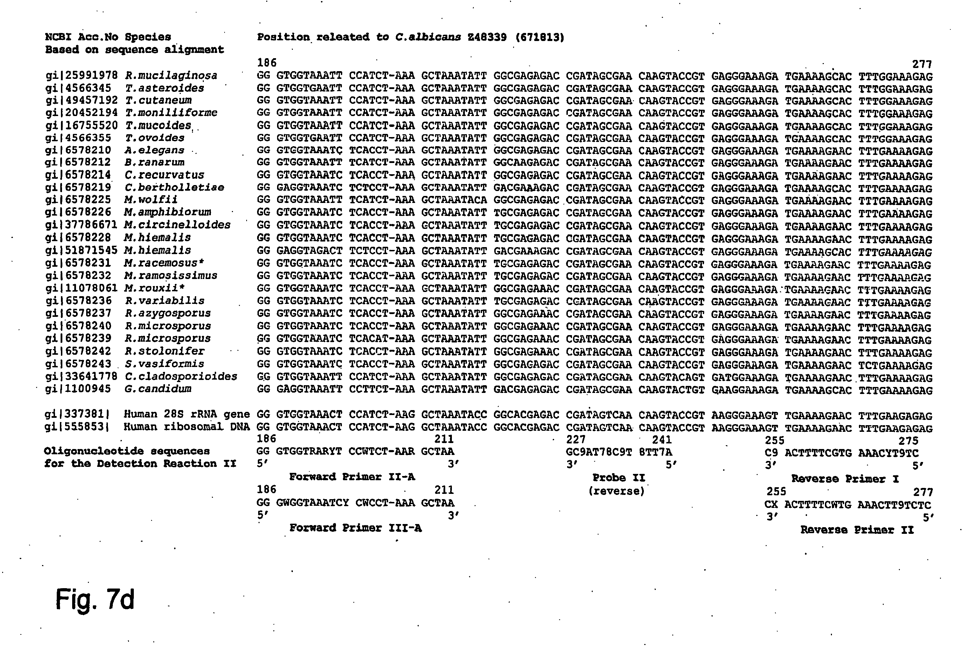

The consensus sequences SEQ ID No. 4 to 7 are derived from the alignments shown in Figs. 6 and 7, whereby for SEQ ID No. 4, 5 and 6 the alignments starting from nucleotide 231 and 196 (related to A. fumigatus, Fig. 6), respectively, were considered. SEQ ID No. 7 is derived from the alignment outline in Fig. 7 starting from nucleotide 152 (related to Candida albicans).

In the nucleic acid sequences provided herein "A" stands for adenosine, "T" for thymidine, "G" for guanosine, "C"' for cytosine, "N" for adenosine, thymidine, guanosine or cytosine, "V" for adenosine, guanosine or cytosine, "D" for adenosine, thymidine or guanosine, "B" for thymidine, guanosine or cytosine, "H" for adenosine, thymidine or cytosine, "W" for adenosine or thymidine, "S" for guanosine or cytosine, "K" for thymidine or guanosine, "M" for adenosine or cytosine, "Y" for thymidine or cytosine and "R" for adenosine or guanosine.

The use of primers derived from a sequence alignment of more than one nucleotide sequence allows for the "simultaneous detection" of at least one fungus in a sample because these primers are able to bind to the DNA of a number of fungi. However, it is also possible to detect in a first nucleic acid amplification reaction with a first primer pair, a group of fungi, and in at least one further nucleic acid amplification reaction at least one other group of fungi. However, it is preferred to detect the presence of at least one fungus of one or more genera with a maximum of five, preferably with a maximum of four, more preferably with a maximum of three, most preferably with a maximum of two, especially with a maximum of one, nucleic acid amplification step.

"Providing a sample" refers to methods known in the state of the art which may be employed to provide such samples, which potentially comprise at least one fungus. Therefore, also methods for the extraction and purification of nucleic acids from said samples are meant by "providing". Such extraction methods are disclosed, for instance in Sambrook J. and Russell T.B. (2001) "Molecular cloning - A laboratory manual" Cold Spring Harbor Laboratory Press NY. Of course, for certain nucleic acid amplification techniques (e.g. in situ PCR), the isolation of a nucleic acid from a sample is not necessary.

The term."sample" includes all types of samples which may be infected by at least one fungus. However, preferred samples are clinical samples (e.g. blood serum, tissues) of, for instance, immunosuppressed patients or patients suspected to be infected with a fungus. The sample may be prepared before contacting it with the primers by standard laboratory methods (e.g. homogenisation and/or fractionation of the sample, purification or precipitation of the nucleic acid) or used directly for the nucleic acid amplification. The method according to the present invention may also be used to detect at least one fungus in a sample e.g. to test its sterility (e.g. solutions employed in medicine, like infusions). When analyzing solid material the sample may be obtained by washing said material with a fungus-free solution.

According to the present invention "derived from" means that the primers or probes, which may comprise at least 10, preferably at least 15, nucleotides, are directly deduced from the nucleotide sequences (alignments or individual sequences) disclosed herein (SEQ ID No. 1 to 7) and are at least 80%, at least 90%, at least 95%, at least 98%, at least 99% or 100% identical to them. Therefore these primers substantially correspond to a region of the designated nucleotide sequence. These primers can be obtained by simple chemical synthesis (e.g. Caruthers, M.H. (1983) Tetrahedron Lett. 24:245-248) or by enzymatic or chemical cleavage of a nucleic acid sequence comprising a primer sequence. As such, the primers may represent either a sense or an antisense orientation of the original polynucleotide.

Whether any two nucleic acid molecules have nucleotide sequences that are "identical" to a certain degree ("% identity") can be determined using known computer algorithms such as the "FAST A" program, using for example, the default parameters as in Pearson et al. (1988) PNAS USA 85: 2444 (other programs include the GCG program package (Devereux, J., et al., Nucleic Acids Research (1984) Nucleic Acids Res., 12, 387-395), BLASTP, BLASTN, FASTA (Atschul, S.F., et al., J Molec Biol 215: 403 (1990); Guide to Huge Computers, Martin J. Bishop, ed., Academic Press, San Diego, 1994, and Carilloet al, (1988)SIAM J Applied Math 48 : 1073). For instance, the BLAST tool of the NCBI database can be used to determine identity. Other commercially or publicly available programs include, DNAStar "MegAlign" program (Madison,WI) and the University of Wisconsin Genetics Computer Group(UWG) "Gap" program (Madison, WI)). Percent homology or identity of nucleic acid molecules can be determined, for example, by comparing sequence information using a GAP computer program (e.g. Needleman et al., (1970) J. Mol. Biol. 48:443, as revised by Smith and Waterman (1981) Adv. Appl. Math. 2:482). Briefly, the GAP program defines similarity as the number of aligned symbols (i.e., nucleotides or amino acids) which are similar, divided by the total number of symbols in the shorter one of the two sequences. Default parameters for the GAP program can include : (1) a unary comparison matrix (containing a value of 1 for identities and for non-identities) and the weighted comparison matrix of Gribskov et al. 14:6745, as described by Schwartz and Dayhoff, eds., ATLAS OF PROTEIN SEQUENCE AND STRUCTURE, National Biomedical Research Foundation, pp. 353-358 (1979); (2) a penalty of 3.0 for each gap and an additional 0.10 penalty for each symbol in each gap; and (3) no penalty for end gaps.

According to the present invention it is also possible to use more than one primer for the detection of at least one fungus in a sample. The primer pairs and in particular their forward and reverse primer may be derived not only from one aligned sequence (e.g. SEQ ID No. 1) but also from combinations of two or more of such sequences (e.g. SEQ ID No. 1 and 4, 2 and 7, 3 and 5). The combination of different primers and primer pairs will allow e.g. to determine the presence of a certain group of fungi in a sample. Furthermore, such combinations may also broaden the number of fungi to be detected.

Typically, primers deduced from a nucleic acid sequence (alignment sequence or single sequence) may be designed following general rules known to the person skilled in the art (e.g. Dieffenbach, C.W., et al., General Concepts for PCR Primer Design, in PCR Primer, A Laboratory Manual, Dieffenbach, C.W, and Dveksler, G.S., Ed., Cold Spring Harbor Laboratory Press, New York, 1995, 133-155; Innis, M.A., and Gelfand, D.H., Optimization of PCRs, in PCR protocols, A Guide to Methods and Applications, Innis, M.A., Gelfand, D.H., Sninsky, J.J., and White, T.J., Ed., CRC Press, London, 1994, 5-11; Sharrocks, A.D., The design of primers for PCR, in PCR Technology, Current Innovations, Griffin, H.G., and Griffin, A.M, Ed., CRC Press, London, 1994, 5-11; Suggs, S.V., et al., Using Purified Genes, in ICN-UCLA Symp. Developmental Biology, Vol. 23, Brown, D.D. Ed., Academic Press, New York, 1981, 683; Kwok, S., et al., Effects of primer-template mismatches on the polymerase chain reaction: Human Immunodeficiency Virus 1 model studies. Nucleic Acids Res. 18:999-1005, 1990). The primers employed in a method according to the present invention may be partly degenerated or contain no degenerated nucleotide position. Therefore, suited regions on the nucleic acid sequences may be selected to design primers with a low number of degenerated nucleotide positions, wherein suited primers comprise less than 80%, less than 70%, less than 60%, less than 50%, less than 40%, less than 30%, less than 20%, less than 15%, less than 10%, less than 5%, or no degenerated nucleotide positions. Instead of nucleotides in the degenerated nucleotide positions, inosine may be used. However, primers without any degenerated nucleotide position are preferably used.

According to a preferred embodiment of the present invention the at least one fungus is a member of a fungus genus selected from the group consisting of Aspergillus, Candida, Fusarium, Trichosporon, Mucor, Rhizopus, Acremonium, Cryptococcus, Scedosporium, Alternaria, Histoplasma, Penicillium, Bipolaris, Cladosporium, Malassezia, Saccharomyces, Rhodotorula, Apophysomy- . ces, Basidiobolus, Cokeromyces, Cunninghamella, Mortierella, Rhizomucor, Saksenaea, Geotrichum and combinations thereof.

Members of these fungal genera are well-known pathogens in clinical practice, and some of them are responsible for severe fungal infections in patients, in particular in immunocompromised or immunodeficient patients. In order to apply to said patients an appropriate therapy it is of major importance to unequivocally determine the presence of a fungus in said patient.

The at least one fungus is preferably selected from the group consisting of Aspergillus flavus, Aspergillus fumigatus, Aspergillus nidulans, Aspergillus niger, Aspergillus terreus, Aspergillus candidus, Aspergillus clavatus, Aspergillus ochraceus, Aspergillus penicillioides, Aspergillus ustus, Aspergillus versicolor, Candida albicans, Candida cylindracea, Candida dubliniensis, Candida famata, Candida glabrata, Candida guilliermondii, Candida inconspicua, Candida kefyr, Candida krusei, Candida lambica, Candida lusitaniae, Candida membranaefaciens, Candida norvegensis, Candida parapsilosis, Candida pelliculosa, Candida rugosa, Candida sake, Candida tropicalis, Candida utilis, Candida allociferii, Candida colliculosa, Candida lipolytica, Candida zeylanoides, Fusarium oxysporum, Fusarium proliferatum, Fusarium solani, Fusarium verticillioides, Acremonium strictum, Trichosporon asahii, Trichosporon cutaneum, Trichosporon inkin, Trichosporon moniliiforme, Trichosporon asteroides, Trichosporon ovoides, Trichosporon mucoides, Mucor circinelloides, Mucor hiemalis, Mucor mucedo, Mucor racemosus, Mucor ramosissimus, Mucor amphibiorum, Mucor rouxii, Rhizopus oryzae, Rhizopus azygosporus, Rhizopus microsporus, Rhizopus stolonifer, Absidia corymbifera, Cryptococcus albidus, Cryptococcus laurentii, Cryptococcus neoformans, Scedosporium apiospermum, Scedosporium proliferans, Alternaria alternata, Histoplasma capsulatum, Penicillium chrysogenum, Cladosporium cladosporioides, Cladosporium oxysporum, Blastoschizomyces capitatus, Malassezia furfur, Malassezia sympodialis, Saccharomyces cerevisiae, Rhodotorula mucilaginosa, Apophysomyces elegans, Basidiobolus ranarum, Cokeromyces recurvatus, Cunninghamella bertholletiae, Mortierella wolfii, Saksenaea vasiformis, Geotrichum candidum and combinations thereof.

However, the at least one fungus is most preferably selected from the group consisting of Aspergillus flavus, Aspergillus fumigatus, Aspergillus niger, Aspergillus terreus, Candida albicans, Candida dubliniensis, Candida glabrata, Candida guilliermondii, Candida kefyr, Candida krusei, Candida lusitaniae, Candida parapsilosis, Candida tropicalis, Fusarium oxysporum, Fusarium solani, Trichosporon asahii, Trichosporon cutaneum, Trichosporon inkin, Mucor mucedo, Rhizopus oryzae, Cryptococcus albidus, Cryptococcus laurentii and Cryptococcus neoformans, which represent the most wide spread and the clinically most relevant fungi. Of course the clinical relevance of a single fungus varies from region to region.

The sample of step a) may be further contacted with at least one nucleic acid probe which sequence is derived from SEQ ID No. 1, SEQ ID No. 2, SEQ ID No. 3, SEQ ID No. 4, SEQ ID No. 5, SEQ ID No. 6, SEQ ID No. 7 or combinations thereof and which sequence is located between the forward and the reverse primer derived from SEQ ID No. 1, SEQ ID No. 2, SEQ ID No. 3, SEQ ID No. 4, SEQ ID No. 5, SEQ ID No. 6 or SEQ ID No. 7 thereof.

In order to unequivocally determine the presence of a fungus in a sample, a nucleic acid probe which is able to bind to the nucleic acid amplification product is added to the sample prior to or after the application of the nucleic acid amplification technique. Since the nucleic acid probe has to bind to the amplification product, it is necessary that said probe is derived from a sequence stretch of SEQ ID No. 1, SEQ ID No. 2, SEQ ID No. 3, SEQ ID No. 4, SEQ ID No. 5, SEQ ID No. 6 or SEQ ID No. 7 which is located between the forward and reverse primer. The nucleic acid probe may be derived from SEQ ID No. 1, SEQ ID No. 2, SEQ ID No. 3, SEQ ID No. 4, SEQ ID No. 5, SEQ ID No. 6 or SEQ ID No. 7 or from the complementary strand of said sequences.

The at least one nucleic acid probe is preferably conjugated to a dye, preferably to a fluorescent dye, and optionally to a quencher.

In order to detect the binding of the probe to the amplification product, said probe is labelled with a dye. Depending on the dye or dye/quencher combination used, the amplified product may be detected during or after the application of the nucleic acid amplification technique.

According to a preferred embodiment of the present invention, the nucleic acid amplification technique is a polymerase chain reaction technique, which may be selected from the group consisting of real-time PCR, preferably TaqMan PCR, quantitative PCR, nested PCR, asymmetric PCR, multiplex PCR, inverse PCR, rapid PCR and combinations thereof.

Due to the specificity of these polymerase chain reaction techniques, all of them are suited to be used in a method according to the present invention. However, the use of real-time PCR is preferred, because this technique is able to provide results in a relatively short time.

Advanced technologies, such as real-time quantitative PCR (RQ-PCR) eliminate the post-amplification handling steps. They have the potential to not only substantially increase the sensitivity of the PCR, but also to provide the means to monitor response to therapy by measuring fungal, burden at any given point in time. A considerable amount of data is available on the detection of IFIs using quantitative PCR approaches. However, many published assays only permit detection of individual fungal species (Costa et al., 2001) (Loeffler et al., 2000). To date, a few RQ-PCR methods covering either multiple Candida species (Maaroufi et al., 2004), Aspergillus species (Sanguinetti et al., 2003) or other mold infections (Pham et al., 2003) are available. A recently published approach permits the detection of a broad spectrum of fungi, however, its application is limited to the light cycler technology (Jordanides et al., 2005).

The amplified nucleic acid product is preferably additionally tagged for detection by a technique selected from the group consisting of DNA-tagging by random-priming, DNA-tagging by nick-translation, DNA-tagging by polymerase chain reaction, oligonucleotide tailing, hybridization, tagging by kinase activity, fill-in reaction applying Klenow fragment, photobiotinylation and combinations thereof.

Due to a tagging of the PCR product, this product can easily be visualised by an additional further step involving for instance gel electrophoresis.

According to a preferred embodiment of the present invention the amplified nucleic acid product is detected by gel electrophoresis, Southern-blot, photometry, chromatography, colorimetry, fluorography, chemiluminescence, autoradiography, detection by specific antibody and combinations thereof.

All these methods turned out to be suitably used in order to detect the amplification of a nucleic acid sequence.

The forward and the reverse primer of the at least one primer pair consists preferably of 15 to 40, preferably of 16 to 35, more preferably of 17 to 30, nucleotides.

The size of the primers used in a method according to the present invention can vary. When sequence specific primers are designed, it has to be considered that the primer length influences the specificity of the primer as well as the melting temperature of the respective primer-DNA complex. It turned out that especially primers with 15 to 40 nucleotides are valuable.

The at least one nucleic acid probe consists preferably of 9 to 40, preferably of 10 to 35, more preferably of 12 to 30, nucleotides.

According to a preferred embodiment of the present invention, the primers of the at least one primer pair and/or the at least one nucleic acid probe comprise at least one LNA (locked nucleic acid) nucleotide.

In order to increase the melting temperature of primers and probes one or more of the nucleotides of said nucleic acids may be substituted or modified with corresponding nucleotides and/or substances. LNA nucleotides are preferably used to increase said temperature. LNA is a bicyclic nucleic acid where a ribonucleoside is linked between the 2'-oxygen and the 4'-carbon atoms with a methylene unit (see e.g. EP 1 013 661). Locked nucleic acids (LNA) are a class of conformationally restricted oligonucleotide analogues. The main mechanism for LNA oligos binding plasmid DNA is demonstrated to be by strand displacement. LNA oligos are more stably bound to plasmid DNA than similar peptide nucleic acid (PNA) 'clamps' for procedures such as particle mediated DNA delivery (gene gun). LNA oligos can bind to DNA in a sequence-specific manner so that binding does not interfere with plasmid conformation or gene expression. LNA oligonucleotides exhibit unprecedented thermal stabilities towards complementary DNA and RNA, which allow excellent mismatch discrimination. The high binding affinity of LNA oligos allows for the use of short probes in antisense protocols and may be used in any hybridization assay that requires high specificity and/or reproducibility, e.g., dual labelled probes, in situ hybridization probes, molecular beacons and PCR primers. Furthermore, LNA offers the possibility to adjust Tm values of primers and probes in multiplex assays. Each LNA base addition in an oligo increases the Tm by approximately 8°C. The synthesis and incorporation of LNA bases can be achieved by using standard DNA synthesis chemistry.

Primers and probes to be used in a method according to the present invention may comprise one or more LNA bases in order to approximate the melting temperatures of the primer pair and optionally of the probe. The temperature of LNA primers and probes may be calculated by methods known in the art (Tolstrup N, et al. (2003) Nucleic Acids Res. 31:3758-62; McTigue PM. (2004) Biochemistry. 43:5388-405).

The forward primers of the primer pair 5'-TAAAGCTAAATAYYGGCCRGAGA-3' (SEQ ID No. 11), 5'-GGGTGGTRARYTCCWTCTAARGCTAA-3' (SEQ ID No. 13) and 5'-GGGWGGTAAATCYCWCCTAAAGCTAA-3' (SEQ ID No. 14).

The reverse primers of the primer pair are 5'-CTTTYCAAAGTGCTTTTCATC-3' (SEQ ID No. 19) and 5'-CTCTTTTCAAAGTWCTTTTCAYC-3' (SEQ ID No. 20).

The primer pair (e.g. one forward and one reverse primer) has to be chosen in order to allow for the amplification of a nucleic acid fragment which can be detected with a method as outlined above. In order to balance the melting temperatures of the primers used some of the reverse primers may comprise LNA modified nucleotides as outlined above. In particular one or both of the nucleotides in parenthesis of the reverse primers 5'-CT(T)TYCAAAGTGCTTTTCA(T)C-3' (SEQ ID No. 19) and 5'-CTCT(T)TTCAAAGTWCTTTTCA(Y)C-3' (SEQ ID No. 20) may be modified with corresponding LNA bases. In order to avoid cross reactions with human DNA it turned out that especially those regions of the primers may suitably be modified with LNA bases. Consequently, the LNA modification concerning the 3' end of SEQ ID No. 19 and 20 are most preferably performed (see e.g. Fig. 6b).

The nucleic acid probes are 5'-ACTTGTGCGCTATCG-3' (SEQ ID No. 26) and 5'-ACTTGTTCGCTATCG-3' (SEQ ID No. 35).

The nucleic acid probe is selected, of course, so that it can specifically bind to a nucleic acid fragment amplified with a primer pair.

For instance, fungi of the genera Aspergillus and Candida may be detected when the primers SEQ ID. No. 10 and 23, optionally in combination with the probe SEQ ID No. 24 are used. Fungi, which are members of various genera, may be detected, e.g., if SEQ ID No. 11 or 12 are used in combination with SEQ ID No. 19 or 20, if one of SEQ ID No. 13 to 16 is used in combination with SEQ ID No. 19 or 20.

The primers and probes according to the present invention may comprise instead of nucleotides LNA nucleotides. For instance, 5'-ACT(T)GT(G)CG(C)TA(T)CG-3' (SEQ ID No. 26), 5'-CT(T)TYCAAAGTGCTTTTCA(T)C-3' (SEQ ID No. 19), 5'-CTCT(T)TTCAAAGTWCTTT TCA(Y)C (SEQ ID No. 20), 5'-A(C)TT(G)T(T)C(G) (C)TA(T)CG-3' (SEQ ID No. 26), 5'-CTT(G)TK(C) (G)CT(A)TCGGT-3' (SEQ ID No. 33), 5'-TAC(T)T(G)TK(C)(G)CT(A)TCG-GT-3' (SEQ ID No. 34) and 5'-ACT(T)GT(T)CG(C)TA(T)CG-3' (SEQ ID No. 35), wherein the nucleotides in parenthesis may be all or in part substituted with corresponding LNA nucleotides, are preferably employed in a method or kit according to the present invention. In particular, it is advantageous to substitute nucleotides with LNA nucleotides in those regions of the primers which are, when aligned to human DNA sequences, differing from the human DNA. This allows on one hand to increase the melting temperature of the primer-target pair and on the other hand to bind more selectively to the target rather than unspecifically to the human DNA.

At least one fungus of the genus Candida can be detected when 5'-GGGTGGTAAATTCCATCTAARGCTAA-3' (SEQ ID No. 8) as forward primer, 5'-AAGTTCTTTTCATCTTTCCWTCACWGT-3' (SEQ ID No. 17) or 5'-CTTTTCAAAGTKCTTTTCARC-3' (SEQ ID No. 21) as reverse primer and optionally 5'-CTTGTKCGCTATCGGTCTCTSGCCA-3' (SEQ ID No. 24) as nucleic acid probe is contacted with the sample.

The use of these primers and this probe allows for the specific detection of fungi of the genus Candida, if present, in a sample.

At least one fungus of the genus Aspergillus can be detected when 5'-GGGTGGTAAATTTCATCTAAAGCTAA-3' (SEQ ID No. 9) as forward primer, 5'-AAGTTCTTTTCATCTTTCGATCACTCT-3' (SEQ ID No. 18) or 5'-CTTTTCAAAGTGCTTTTCATC-3' (SEQ ID No. 22) as reverse primer and optionally 5'-CTTGTKCGCTATCGGTCTCTSGCCA-3' (SEQ ID No. 24) as nucleic acid probe is contacted with the sample.

Fungi of the genus Aspergillus are preferably detected in a sample by using the above mentioned primers and probe.

At least one fungus of the genera Aspergillus and Candida can be detected when 5'-GGGTGGTAAATTYCATCTAARGCTAA-3' (SEQ ID No. 10) as forward primer, 5'-CTTTYCAAAGTGCTTTTCATC-3' (SEQ ID No. 19) and 5'-CTCTTTTCAAAGTWCTTTTCAYC-3' (SEQ ID No. 20) or 5'-AAGTKCTTTTCATCTTTCSWTCACWST-3' (SEQ ID No. 23) as reverse primer and optionally 5'-CTTGTKCGCTATCGGTCTCTSGCCA-3' (SEQ ID No. 24) as nucleic acid probe is contacted with the sample.

The use of said primer probes in a nucleic acid amplification method allow for the detection of fungi of the genera Aspergillus and Candida in a sample. Especially the clinically relevant fungi (see e.g. Table 2) can be detected using said primers/probe.

According to a preferred embodiment of the present invention a nucleic acid as positive control and a positive control specific primer pair and optionally a positive control specific nucleic acid probe derived from said nucleic acid is added to the sample after method step a), wherein said nucleic acid does not result in a nucleic acid amplification product when contacted and amplified with a primer pair derived from SEQ ID No. 1, SEQ ID No. 2, SEQ ID No. 3, SEQ ID No. 4, SEQ ID No. 5, SEQ ID No.6, SEQ ID No. 7 or combinations thereof, and wherein the primer pair derived from said nucleic acid does not result in a nucleic acid amplification product when contacted with a sample free of any fungus.

The provision of a positive control in the method according to the present invention allows for monitoring the performance of the nucleic acid amplification technique. When using positive controls as well as positive control specific primer pairs and/or probes, the cross reactivity between the sample and the positive control as well as their respective primer pairs has to be considered. Therefore, the primer pair which is used for the detection of fungal DNA may not be able to amplify an amplification product with the positive control.

The nucleic acid added as positive control is human or phocine herpesvirus DNA.

In order to monitor the performance of the nucleic acid amplification technique a human or phocine herpesvirus DNA as template is added to the sample. Although in samples obtained from human individuals human DNA may already be contained, additional DNA is preferably added.

The positive control specific primer pair preferably comprises a forward primer 5'-TGAGTATGCCTGCCGTGTGA-3' (SEQ ID No. 27), a reverse primer 5'-ACTCATACACAACTTTCAGCAGCTTAC-3' (SEQ ID No. 28) and a positive control specific nucleic acid probe 5'-CCATGTGACTTTGTCACAGCCCAAGATAGTT-3' (SEQ ID No. 29), when human DNA is added to the sample, and a forward primer 5'-GGGCGAATCACAGATTGAATC-3' (SEQ ID No. 30), a reverse primer 5'-GCGGTTCCAAACGTACCAA-3' (SEQ ID No. 31) and optionally a positive control specific nucleic acid probe 5'-TTTTTATGTGTCCGCCACCATCTGGATC-3' (SEQ ID No. 32), when phocine herpesvirus DNA is added to the sample.

These primers specifically bind to the human β2-MG (micro-globulin) and the phocine herpesvirus gB gene, respectively. These primers bind specifically to human and phocine herpesvirus and not to fungal DNA.

Another aspect of the present invention relates to the use of a method as outlined above for the detection of at least one fungus of the genus Aspergillus, Candida, Fusarium, Trichosporon, Mucor, Rhizopus, Acremonium, Cryptococcus, Scedosporium Alternaria, Histoplasma, Penicillium, Bipolaris, Cladosporium, Malassezia, Saccharomyces, Rhodotorula, Apophysomyces, Basidiobolus, Cokeromyces, Cunninghamella, Mortierella, Rhizomucor, Saksenaea and/or Geotrichum in a sample (in particular those which can be detected with the method according to the present invention).

Yet another aspect of the present invention relates to a kit for the simultaneous detection of at least one fungus in a sample comprising:
- forward primers 5'-TAAAGCTAAATAYYGGCCRGAGA-3' (SEQ ID No. 11), 5'-GGGTGGTRARYTCCWTCTAARGCTAA-3' (SEQ ID No. 13) and 5'-GGGWGGTAAATCYCWCCTAAAGCTAA-3' (SEQ ID No. 14),
- reverse primers 5'-CTTTYCAAAGTGCTTTTCATC-3' (SEQ ID No. 19) and 5'-CTCTTTTCAAAGTWCTTTTCAYC-3' (SEQ ID No. 20),
- nucleic acid probes, in particular labelled nucleic acid probes 5'-ACTTGTGCGCTATCG-3' (SEQ ID No. 26) and 5'-ACTTGTTCGCTATCG-3' (SEQ ID No. 35),
- optionally a nucleic acid as positive control and a positive control specific primer pair and nucleic acid probe as defined in any one of claims 10 to 12.

The present invention is further illustrated by the following figures and examples.

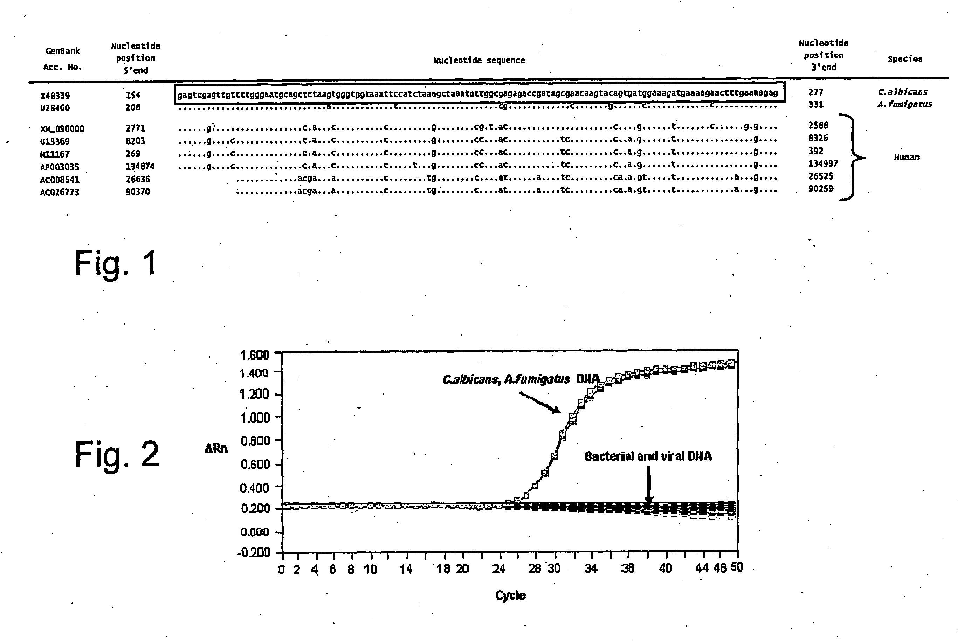

Fig. 1 shows a multiple alignment of fungal 28S rRNA gene sequences with homologous human DNA regions. Comparison of sequences targeted by the RQ-PCR assay presented between selected fungal species (*A.fumigatus, C.albicans*) and homologous human DNA sequences, as revealed by alignment using the 'ClustalW' software. Identical nucleotides are shown as dots, differences are specified.
Fig. 2 shows the amplification of bacterial and viral DNA by the pan-AC detection system. The absence of cross-reactivity between the primer/probe system of the pan-AC fungus assay with clinically relevant non-fungal pathogens was documented by the investigation of DNA derived from more than 20 different bacterial or viral species. *C.albicans* and *A.fumigatus* gDNA (genomic DNA) were used as positive controls of amplification by the pan-AC assay.

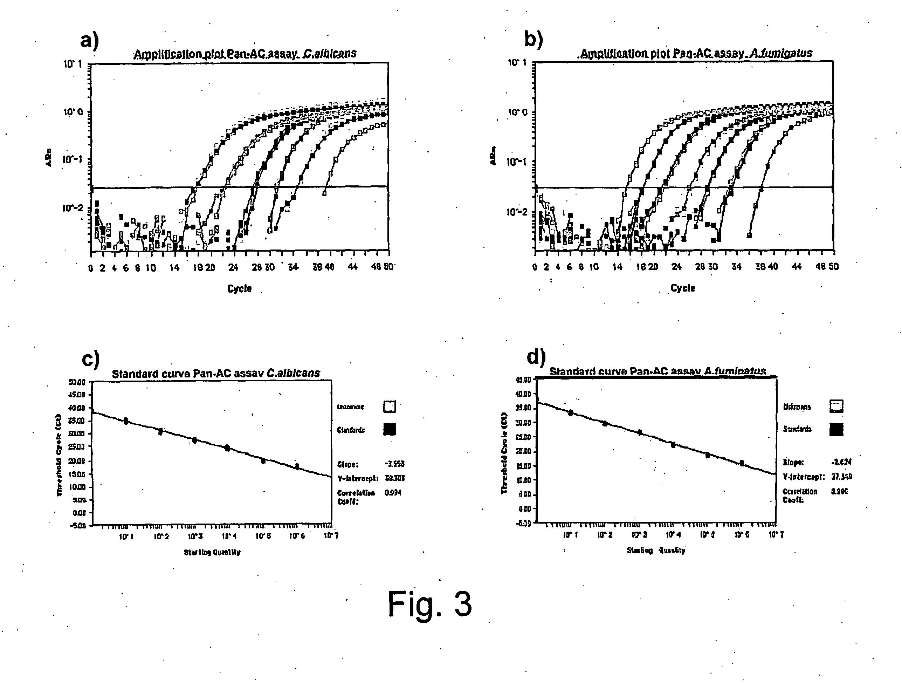

Fig. 3 shows standard curves for quantitative fungus analysis by the pan-AC assay. The upper panels show amplification plots of serial logarithmic dilutions of fungal gDNA derived from *C.albicans* (a) and *A.fumigatus* (b). The lower panels (c, d) reveal the corresponding standard curves generated on the basis of the amplification profiles displayed. In view of the great similarity between the amplification profiles of all fungal species covered by the pan-AC assay, the standard curves shown are applicable to the quantification of any *Candida* or *Aspergillus* species detected.

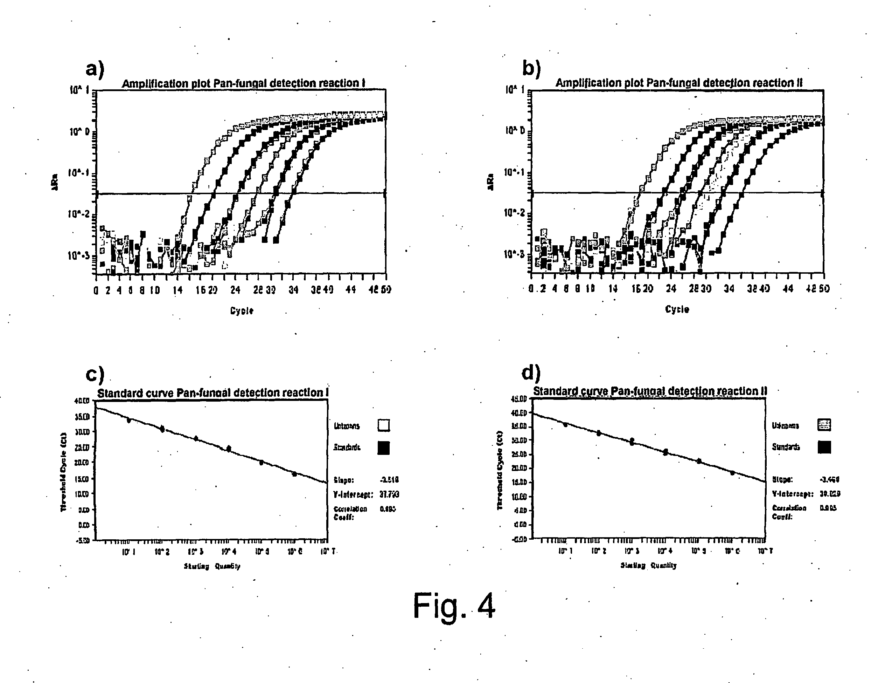

Fig. 4 shows standard curves for quantitative fungus analysis by the Pan-fungal assay. The upper panels show amplification plots of serial logarithmic dilutions of fungal gDNA derived from *A.fumigatus* (a) amplified by the Pan-fungal detection reaction I and *C.albicans* (b) amplified by the Pan-fungal detection reaction II. The lower panels (c, d) reveal the corresponding standard curves generated on the basis of the amplification profiles displayed. In view of the great similarity between the amplification profiles of all fungal species covered by the Panfungal assay, the standard curves shown are applicable to the
quantification of any *Candida, Aspergillus,* and other fungal species within the detection spectrum of the assay.

Fig. 5 shows alignments of fungi of the genera Aspergillus and Candida (Table 2). The positions of the nucleotides of the nucleic acid sequences are related to Candida albicans (Acc.No. Z48339) .

Fig. 6 shows alignments of fungi of several genera (Table 4). The positions of the nucleotides of the nucleic acid sequences are related to Aspergillus fumigatus (Acc.No. U28460). LNA modified nucleotides used in the oligonucleotide sequences:
X = C-LNA or T-LNA; 7 = C-LNA; 8 = G-LNA; 9 = T-LNA.

Fig. 7 shows alignments of fungi of several genera (Table 5). The positions of the nucleotides of the nucleic acid sequences are related to Candida albicans (Acc.No. Z48339). LNA modified nucleotides used in the oligonucleotide sequences: X = C-LNA or T-LNA; 7 = C-LNA; 8 = G-LNA; 9 = T-LNA.

### EXAMPLES:

### Example 1:

### 1.1. Materials and Methods:

### 1.1.1. Fungal strains, bacteria and virus isolates

Fungal strains for PCR testing were obtained from the American Type Culture Collection (ATCC, Rockville, USA): Aspergillus fumigatus (ATCC 36607), Aspergillus niger (ATCC 10535), Candida albicans (ATCC 14053), Candida dubliniensis (ATCC MYA-646), Candida glabrata (ATCC 2001), Candida krusei (ATCC 6258), Candida, parapsilosis (ATCC 22019), Candida tropicalis (ATCC 750), Scedosporium apiospermum (ATCC 28206), from the German Collection of Microorganisms (DSM, Braunschweig, Germany): Acremonium strictum (DSM 3567; DSM 2399), Alternaria alternata (DSM 62006), Aspergillus candidus (DSM 814), Aspergillus clavatus (DSM 816), Aspergillus flavus (DSM 818), Aspergillus fumigatus (DSM 819), Aspergillus nidulans (DSM 820; DSM 946), Aspergillus niger (DSM 737), Aspergillus terreus (DSM 826; DSM 1958), Aspergillus versicolor (DSM 1943), Candida albicans (DSM 1386), Candida famata (DSM 70590), Candida glabrata (DSM 11226), Candida guillermondii (DSM 70051; DSM 11947), Candida inconspicua (DSM 70631), Candida kefyr (DSM 70073), Candida krusei (DSM 70075), Candida lambica (DSM 70090), Candida lipolytica (DSM 8218), Candida lusitaniae (DSM 70102), Candida membranaefaciens (DSM 70109), Candida norvegensis (DSM 70760), Candida sake (DSM 70763), Candida parapsilosis (DSM 11224), Candida rugosa (DSM 70761), Candida pelliculosa (DSM 70130), Candida tropicalis (DSM 5991), Candida zeylanoides (DSM 70185), Cryptococcus albidus (DSM 70215), Cryptococcus laurentii (DSM 70766), Cryptococcus neoformans (DSM 70219), Fusarium oxysporum (DSM 2018; DSM 12646), Fusarium proliferatum (DSM 840; DSM 764), Fusarium solani (DSM 62413; DSM 1164), Malassezia furfur (DSM 6170; DSM 6171), Mucor mucedo (DSM 809), Rhizopus oryzae (DSM 853), Saccharomyces cerevisiae (DSM 70449) and Trichosporon cutaneum (DSM 70707). Additional control samples were obtained from the Institute of Hygiene and Medical Microbiology, Medical University of Vienna, Austria: Absidia corymbifera, Aspergillus candidus, Aspergillus clavatus, Aspergillus flavus, Aspergillus fumigatus, Aspergillus nidulans, Aspergillus niger, Aspergillus terreus, Aspergillus versicolor, Blastoschizomyces capitatus, Candida.albicans, Candida colliculosa, Candida famata, Candida glabrata, candida guilliermondii, Candida kefyr, Candida krusei, Candida lambica, Candida lipolytica, Candida lusitaniae, Candida norvegensis, Candida parapsilosis, Candida rugosa, Candida pelliculosa, Candida valida, Candida zeylanoides, Cryptococcus laurentii, Cryptococcus neoformans, Rhizopus oryzae, Saccharomyces cerevisiae, Trichosporon asahii and Trichosporon inkin. Prior to DNA extraction, the fungal isolates were grown on Sabouraud dextrose agar: the yeasts at 37°C (Cryptococcus spp. 30°C) for 48 hours and the molds at 30°C for one to seven days. Yeast identification was established by the APID32C test (BioMerieux, Marcy I'Etoile, France) and by micromorphology on rice-agar. Molds were identified based on conventional macro- and microscopic morphological investigation. Suspensions of all fungi (molds, conidia suspension; yeasts, cell suspension) were prepared using an aliquot of cultured fungus resuspended in sterile 0.9% NaCl solution.
In addition, a panel of bacterial and viral pathogens was selected for testing of cross-reactivity. The panel included Enterobacter aerogenes, Eschericha coli, Haemophillus influenzae, Klebsiella pneumoniae, Listeria monocytogenes, Neiseria meningitidis, Pseudomonas aeruginosa, Staphylococcus aureus, Streptococcus pneumoniae, human adenoviruses (AdV), Epstein-Barr virus (EBV), cytomegalovirus (CMV), varicella zoster virus (VZV), herpes simplex virus (HSV) types 1 and 2, human herpes viruses 6, 7 and 8 (HHV6, 7 and 8) and parvovirus B19 (PVB19). DNA isolated from peripheral blood obtained from a number of volunteer donors was used to test for potential cross-reactivity of the fungus assay with human sequences.

### 1.1.2. Sample preparation

a) DNA extraction from fungal suspensions:
   All steps were performed in a laminar flow hood or in specifically dedicated enclosed instruments. Fungal DNA was extracted using the MagNA Pure Compact Instrument (Roche Diagnostic, Switzerland). Due to the composition of fungal cell walls, standard DNA isolation methods are not sufficient for the disruption of fungus particles. Therefore, an additional combined enzymatic-mechanic disruption step has been included prior to DNA extraction. First, fungal suspensions were centrifuged for 10 min at 5000g. The supernatant was discarded and the pellet was resuspended with 450 µl lyticase lysis buffer (50mM Tris (pH 7.6), 1mM EDTA (pH 8.0), 0.2% 2-mercaptoethanol and 1U/100 µl recombinant lyticase (all Sigma, Germany))and incubated at 37°C for 1 hour to generate spheroplasts, as described elsewhere (Loffler et al., 1997) . Thereafter, acid-washed glass beads (710-1180 µm in diameter) were added to the lysate and vortexed vigorously for 2 min to facilitate disruption. Then, 400 µl of the solution were submitted to DNA extraction in the MagNA Pure Compact Instrument using the MagNA Pure Compact Nucleic Acid Isolation kit I (Roche Diagnostics, Germany). In the automated DNA extraction process, the samples were resuspended and stabilized by incubation with a buffer containing chaotropic salt and proteinase K. Subsequently, magnetic glass particles were added to bind the nucleic acid. Unbound substances were removed by several washing steps and DNA was eluted by heating the sample. DNA concentration was determined fluorometrically using the Pi-coGreen® dsDNA Quantification Kit (Molecular Probes, USA).
b) DNA extraction from peripheral blood specimens:
   After hypotonic lysis of the erythrocytes with red cell lysis buffer (RCLB) according to a protocol described previously (Loffler et al., 1997) 10 mM Tris (pH 7.6), 5 mM MgCl₂, 10 mM NaCl (all Sigma), the samples where pelleted and incubated with 450 µl of LLB. The subsequent extraction procedure was as described above (2a).
c) DNA extraction from bacteria and viruses:
   For the isolation of DNA from cultured bacteria and virus stocks, a commercially available kit (QIAamp DNA Mini Kit; QIA-GEN GmbH, Hilden, Germany) was used as recommended by the manufacturer.

### 1.1.3. Target sequence analysis and primer/probe design

Conserved nucleotide sequences of the fungal ribosomal multi-copy genes (18S, 5.8S and 28S) of Aspergillus and Candida species and other fungi of interest were selected and aligned using the multiple sequence alignment with hierarchical clustering (Corpet, 1988) and the BLAST search software at NCBI (http://www.ncbi.nlm.nih.gov/BLAST/).
a) Pan-AC system:
The finally selected detection system targeting the 28S large ribosomal subunit (LSU) was based on the comparison of sequences indicated in Table 2.

**Table 2. GenBank accession numbers of fungal species included in the 28S rRNA gene alignment and covered by the Pan-AC assay.**

| **Fungus species** | **GenBank Accession number** |
|---|---|
| *Aspergillus flavus* | AF027863, U28899 |
| *Aspergillus fumigatus* | U28460, AF109335, Z48340 |
| *Aspergillus nidulans* | AF109337, U29856 |
| *Aspergillus niger* | U28815, AF109344 |
| *Aspergillus terreus* | U28841, AF109340 |
| *Aspergillus versicolor* | AF433108, AF433059 |
| *Candida albicans* | Z48339, X83717, L28817 |
| *Candida cylindracea* | U45823 |
| *Candida dubliniensis* | U57685, AB031020 |
| *Candida famata* | AJ786242 |
| *Candida glabrata* | U44808, Z48341 |
| *Candida guilliermondii* | AF374616, U45709 |
| *Candida inconspicua* | U71062 |
| *Candida kefyr* | AF335978, Y15476 |
| *Candida krusei* | U76347, Z48567 |
| *Candida lambica* | U75726, AF020437 |
| *Candida lipolytica* | AF335977 |
| *Candida lusitaniae* | U44817 |
| *Candida membranaefaciens* | AJ539358 |
| *Candida norvegensis* | Z48568, U75730 |
| *Candida parapsilosis* | AF374609, Z48343 |
| *Candida pelliculosa* | AB126677 |
| *Candida rugosa* | AF374612 |
| *Candida sake* | AJ507662 |
| *Candida tropicalis* | AF267497, Z48346 |
| *Candida utilis* | U73570 |
| *Candida zeylanoides* | U45853 |

Sequences of primer and probe were designed on the basis of nucleotide alignments of the sequences listed in Table 1, in particular in Fig. 5. The resulting consensus sequences (SEQ ID No. 1 to 3) were used to derive primer and probe sequences, which can be employed in a method according to the present invention. In addition, software programs may be used to support the design of said primers and probes (e.g. using the "Primer Express" software (Version 2.0; AB, USA)). The universal probe was labeled with FAM (6-carboxyfluorescein) as reporter molecule at the 5'-end and TAMRA (6-carboxytetramethylrhodamine) as quencher molecule at the 3'-end. The oligonucleotide sequences and their location and the GenBank accession numbers of the corresponding target genes are indicated in Table 3.

**Table 3. 28S rRNA specific primer and probe sequences used in a Pan-AC assay**

| **GenBank Acc. No.** | **Nucleotide position** | **oligonucleotide sequences (5'-3')** | |
|---|---|---|---|
| Z48339 | 186-211 | GGG TGG TAA ATT YCA | forward primer |
| U28460 | 240-265 | TCT AAR GCT AA | |
| Z48339 | 268-242 | AAG TKC TTT TCA TCT | reverse primer |
| U28460 | 322-296 | TTC SWT CAC WST | |
| Z48339 | 240-216 | CTT GTK CGC TAT CGG | probe |
| | | TCT CTS GCC A | |

b) Pan-fungal system:
The size and nucleotide composition limits of the targeted fungal conserved regions can be overcome utilizing locked nucleic acid (LNA) modification of the nucleotides. LNA is a bicyclic RNA analogue, in which the ribose moiety in the sugar-phosphate backbone is structurally constrained by a methylene bridge between the 2'-oxygen and the 4'-carbon atoms. The integration of LNA bases into oligonucleotides changes the conformation of the duplex resulting in stronger binding and thus a higher stability. The integration of LNA monomers therefore allows for shorter oligonucleotides while maintaining the same Tm. Introduction of LNA modification is characterized by high specificity, sensitivity and great discrimination ability. Oligonucleotide combinations for the Pan-fungal detection system were employed based on an alignment of the fungal 28S LSU sequences listed in Tables 4 and 5.

**Table 4. GenBank accession numbers of fungal species included in the 28S rRNA gene alignment and covered by the Pan-fungal detection reaction I.**

| **Fungus species** | **GenBank Accession number** |
|---|---|
| *Acremonium strictum* | AY567000 |
| *Alternaria alternata* | AB100675 |
| *Aspergillus candidus* | U28763 |
| *Aspergillus clavatus* | AY216667 |
| *Aspergillus flavus* | AF027863, U28899 |
| *Aspergillus fumigatus* | AF109335, U28460, Z48340 |
| *Aspergillus nidulans* | AF109337, U29856 |
| *Aspergillus niger* | U28815, AF109344 |
| *Aspergillus ochraceus* | AF433088 |
| *Aspergillus penicillioides* | U81265, U81264 |
| *Aspergillus terreus* | U28841, AF109340 |
| *Aspergillus ustus* | U29791 |
| *Aspergillus versicolor* | AF433108, AF433059 |
| *Bipolaris eleusines* | AF163994 |
| *Cladosporium cladosporioides* | AY342114 |
| *Cladopsorium oxysporum* | AY342116 |
| *Fusarium oxysporum* | AF130373, Y07991 |
| *Fusarium proliferatum* | AJ271213 |
| *Fusarium solani* | AY097318 |
| *Fusarium verticillioides* | U34526 |
| *Histoplasma capsulatum* | AF071950 |
| *Penicillium chrysogenum* | U15475 ' |
| *Scedosporium apiospermum* | AY213623 |

**Table 5. GenBank accession numbers of fungal species included in the 28S rRNA gene alignment and covered by the Pan-fungal detection reaction II.**

| **Fungus species** | **GenBank Accession number** |
|---|---|
| *Absidia corymbifera* | AF113446, AF113445 |
| *Apophysomyceselegans* | AF113450 |
| *Basidiobolus ranarum* | AF113452 |
| *Blastoschizomyces capitatus* | AB083084 |
| *Candida albicans* | Z48339, X83717, L28817 |
| *Candida allociferrii* | AB041003 |
| *Candida colliculosa* | U72156 |
| *Candida cylindracea* | U45823 |
| *Candida dubliniensis* | U57685, AB031020 |
| *Candida famata* | AJ786242 |
| *Candida glabrata* | U44808, Z48341 |
| *Candida guilliermondii* | AF374616, U45709 |
| *Candida inconspicua* | U71062 |
| *Candida kefyr* | AF335978, Y15476 |
| *Candida krusei* | U76347, Z48567 |
| *Candida lambica* | U75726, AF020437 |
| *Candida lipolytica* | AF335977 |
| *Candida lusitaniae* | U44817 |
| *Candida membranaefaciens* | AJ539358 |
| *Candida norvegensis* | Z48568, U75730 |
| *Candida parapsilosis* | AF374609, Z48343 |
| *Candida pelliculosa* | AB126677 |
| *Candida rugosa* | AF374612 |
| *Candida sake* | AJ507662 |
| *Candida tropicalis* | AF267497, Z48346 |
| *Candida utilis* | U73570 |
| *Candida zeylanoides* | U45853 |
| *Cladosporium cladosporioides* | AY342063 |
| *Cokeromyces recurvatus* | AF113454 |
| *Cryptococcus albidus* | AY296054 |
| *Cryptococcus laurentii* | AY315663, AB035054 |
| *Cryptococcus neoformans* | L14067, AF335984 |
| *Cunnunghamella bertholletiae* | AF113459 |
| *Geotrichum candidum* | U40118 |
| *Malassezia furfur* | AF063214 |
| *Malassezia sympodialis* | AY743628 |
| *Mortireella wolfii* | AF113465 |
| *Mucor amphibiorum* | AF113466 |
| *Mucor circinelloides* | AY213710 |
| *Mucor hiemalis* | AF113468, AY706243 |
| *Mucor mucedo* | AF113470 |
| *Mucor racemosus* | AF113471 |
| *Mucor ramosissimus* | AF113472 |
| *Mucor rouxii* | AF157174 |
| *Rhizomucor variabilis* | AF113476 |
| *Rhizopus azygosporus* | AF113477 |
| *Rhizopus microsporus* | AF113480, AF113479 |
| *Rhizopus oryzae* | AY213625 |
| *Rhizopus stolonifer* | AF113482 |
| *Rhodotorula mucilaginosa* | AF485994 |
| *Saksenaea vasiformis* | AF113483 |
| *Trichosporon asahii* | AF105393 |
| *Trichosporon asteroides* | AF075513 |
| *Trichosporon cutaneum* | A749837 |
| *Trichosporon inkin* | AF105396 |
| *Trichosporon moniliiforme* | AF444719 |
| *Trichosporon mucoides* | AF335988 |
| *Trichosporon ovoides* | AF075523 |

Primer and probe design were achieved by aligning the sequences disclosed in tables 4 and/or 5 and as outlined above (for panAC-system). Also the design guidelines for LNA-substituted oligonucleotides (Eurogentec, Belgium) were applied. Both LNA-substituted probes were modified with FAM as reporter molecule at the 5'-end and with Black Hole Quencher-1TM (BHQ-1 TM) (Eurogentec) at the 3'-end. Two of the tested forward primer combinations have shown a comparably good performance in RQ-PCR analysis. Hence, there are sets of alternative forward primers for both detection systems, all of which are suitable for clinical testing. Both reverse primers R-I and R-II, which are identical in both detection systems, were also LNA-substituted for higher specificity. The oligonucleotide sequences and their location, the amplicon length and the GenBank accession numbers of the corresponding target genes are indicated in Table 6.

**Table 6. 28S rRNA specific primer and probe sequences used in a Pan-fungal assay**

| **GenBank Acc. No.** | **Nucleotide position** | **Oligonucleotide sequences (5'-3')** | |
|---|---|---|---|
| U28460 | 208-229 | | forward primer (FI-B) |
| U28460 | 257-279 | | forward primer (FI-A) |
| U28460 | 295-281 | | probe I |
| U28460 | 329-309 | | reverse primer (R-I) |
| Z48339 | 275-255 | | |
| U28460 | 331-309 | | reverse primer (R-II) |
| Z48339 | 277-255 | | |
| Z48339 | 241-227 | | probe II |
| Z48339 | 186-211 | | forward primer (FII-A) |
| Z48339 | 186-211 | | forward primer (FIII-A) |
| Z48339 | 156-177 | | forward primer (FII-B) |
| Z48339 | 158-178 | | forward primer (FIII-B) |

| | | | |
|---|---|---|---|
| * nucleotides in parentheses are preferably substituted with corresponding LNA nucleotides | | | |

### 1.1.4. Real-time PCR

a) Pan-AC assay:
   Pan-AC PCR reactions were set up in a total volume of 25 µl containing 12.5 µl Universal Master Mix [2x conc., incl. ROX-reference dye and uracil N'-glycosylase (UNG)] (AB), 2% of formamide (Merck, Germany), primers F Pan-AC and R Pan-AC (VBC Genomics, Austria), a single pan-Aspergillus/Candida (P Pan-AC) hydrolysis probe, and 5 µl of genomic DNA (gDNA).
b) Pan-fungal assay:
   Pan-fungal PCR reactions were set up in a total volume of 25 µl containing 12.5 µl Universal Master Mix (AB). The detection reaction I included the forward primer F I-A (or F I-B) and Probe I. The detection reaction II included a combination of two forward primers F II-A and F III-A (or F II-B and F III-B) and Probe II. The reverse primers R-I and R-II were used in both detection reactions.
c) PCR amplification:
   The mixture was transferred to 96-well optimal microtiter plates (AB). Amplification was performed on the ABI 7700 Sequence Detection System (AB) using the following cycling parameters: 2 minutes at 50°C (degradation of potentially present contaminating dUTP-containing amplicons by UNG), 10 minutes at 95°C (inactivation of UNG and activation of AmpliTaq Gold DNA polymerase), followed by 50 cycles of 15 seconds at 95°C and 60 seconds at 60°C (amplification of specific target sequences) (Watzinger et al., 2004).

During the amplification process, fluorescence signal emission was measured every 7 seconds within each well. After data collection and analysis, the emitted signals of each of the wells were plotted in separate amplification curves as normalized fluorescence units (ΔRn) on the Y-axis against cycle numbers on the X-axis. The intersection point of these amplification curves with the threshold of signal detection was defined as threshold cycle (CT). For absolute quantification of the fungal DNA, standard curves were prepared using serial logarithmic dilutions of positive control samples, which were plotted on a logarithmic scale on the X-axis against fluorescence intensity on the Y-axis. The theoretical detection limit of the reaction is represented by the Y-intercept, the point at which the standard curve intersects with the ordinate. It indicates the theoretical CT-value obtained in the presence of a single template molecule at the start of PCR. Samples revealing threshold cycles greater than the calculated Y-intercept were by definition regarded as negative for the respective target. For quantification of the samples, the initial copy number of each target was extrapolated by plotting the CT-values of each of the positive samples onto the standard curve.

### 1.1.5. Sensitivity

a) Sensitivity of the RQ-PCR assays:
   To determine the limit of detection for both assays, RQ-PCR tests were performed with serial logarithmic dilutions (across a seven log range between 1 fg and 1 ng) of genomic DNA derived from A.fumigatus and C.albicans as representatives of molds and yeasts.
b) Sensitivity of the Pan-AC and the Pan-fungal systems:
   To determine the overall sensitivity of the Pan-AC and Panfungal assays for the detection of fungal pathogens in PB samples, suspensions with calculated numbers of A.fumigatus conidia and C.albicans cells were prepared. The number of fungal particles was determined microscopically using a counting chamber and adjusted to a concentration of 1-3x1E+6 aspergillus conidia or candida cells/ml. Tenfold serial dilutions starting from 1-3E+5 to 1-3E+0 were prepared and spiked into 1 ml of fresh peripheral blood (PB) obtained from healthy volunteer donors.

DNA extraction was performed according to the protocol outlined above (see 1.1.2.a).

### 1.1.6. Prevention of contamination

To avoid contamination with air-borne spores, each step, including the preparation of reagents, the extraction procedure and PCR set-up, was carried out in a laminar-flow hood using dedicated reagents and technical equipment. All reagents used were molecular biology grade. Additionally, solutions for DNA extraction were passed through sterile 0.2 µm filters (Corning®, Corning Incorporated, Acton, USA). PCR reagents were aliquotted into single-use sterile tubes and stored at temperatures recommended by the manufacturer.

Moreover, to reduce the risk of false positive results due to contamination with spurious PCR products, all PCR reactions were performed by partially substituting the nucleotide dTTP by dUTP. Prior to amplification, a digestion step with UNG was performed (see above, item 1.1.4.) to digest any contaminating PCR product, if present. In order to prevent digestion of newly synthesized PCR product, the enzyme was inactivated by heat denaturation before the start of amplification.

The routine use of UNG is highly effective, nevertheless each assay included multiple negative controls as described earlier (Watzinger et al., 2004), to prevent false positive test results.

### 1.1.7. Controls

a) Negative controls:
   A number of precautions were taken to control the occurrence of false positive results. To test for the presence of contaminants during the nucleic acid extraction process and for the generation of non-specific amplification products under the assay conditions used, non-homologous templates [no-amplification controls (NACs) - blood from healthy volunteers in the present setting] were co-extracted in each extraction run and amplified in parallel with all samples. Every RQ-PCR test performed also included number of controls lacking template [no template controls (NTCs), PCR water] to check for the occurrence of contamination during PCR set-up.
b) Positive controls
   In order to document efficient nucleic acid extraction and the absence of enzyme inhibitors in the template preparation, various controls were included:

### i) Internal controls

In peripheral blood samples, a single-copy housekeeping gene (beta-2-microglobulin (β2-MG)) (Watzinger et al., 2004) was amplified in separate reactions in parallel to the fungal targets. Sequences of β2-MG primers and probe are indicated in Table 7.

**Table 7. Primer and probe sequences of positive controls used in a Pan-fungal assay**

| | **GenBank Acc. No.** | **Nucleotide position** | **Oligonucleotide sequences (5'-3')** | |
|---|---|---|---|---|
| ß2-MG (human) | M17987 | 343-362 | | forward primer |
| | M17987 | 447-421 | | reverse primer |
| | M17987 | 364-394 | | probe |
| gB gene (phocine herpesvirus, PPHV) | Z68147 | 267-287 | | forward primer |
| | Z68147 | 337-355 | | reverse primer |
| | Z68147 | 305-332 | | probe |

For largely cell-free human specimens, such as plasma, serum or cerebrospinal fluid, the presence of enzyme inhibitors was controlled by spiking of a defined concentration of the phocine herpes virus PHHV into the sample prior to nucleic acid extraction, as described earlier (Watzinger et al., 2004).

### ii) External controls

Blood samples from healthy individuals were spiked with low copy numbers of *A. fumigatus* and *C. albicans.* These samples were processed and amplified in parallel to the samples under investigation.

### 1.2. Results:

### 1.2.1. Sequence analysis and detection system design:

Based on multiple gene alignment of the species of interest (see Table 1 and Table 2), it was possible to identify a highly conserved region spanning less than 150 bp in length optimally fitting the requirements of real-time PCR analysis.
The region is located within the 28S large ribosomal subunit, clustered between coordinates 154 and 277 from the 5'-end of these genes (the numbers are comparable to the primary sequence of the C.albicans 28S rRNA gene, GenBank accession number Z48339). As indicated in Tables 3 to 6, some of the selected primer sequences display a degenerated code. This was advantageous for sensitive detection of fungal species differing from others at single nucleotide positions.

The selection and optimal concentration of primers and probes were assessed in serial tests, in which several combinations of probe, forward and reverse primers were investigated at concentrations ranging from 50-900 nM. The described RQ-PCR assays for fungus detection and for the amplification of internal controls had been adapted to work under identical cycling conditions to render the molecular diagnostic work more economic.

### 1.2.2. Specificity of the Pan-AC assay:

The ability of the Pan-AC system to detect all fungus species of interest was determined by testing DNA derived from cultures of reference strains including A.fumigatus, A.flavus, A.niger, A.terreus, A.versicolor, A.nidulans, C.albicans, C.glabrata, C.krusei, C.tropicalis, C.parapsilosis, C.guilliermondii, C.kefyr, C.lusitaniae, C.dubliniensis and other Candida species, as indicated in Table 1. Target sequences of all species were amplified successfully and with nearly identical amplification efficiencies, displaying CT differences of < 0.7.

### 1.2.3. Specificity of the Pan-fungal assay:

To determine the potential of the Pan-fungal assay to specifically detect a variety of pathogenic fungi, more than 50 different species representative of all clinically relevant fungus genera were repeatedly tested. It was possible to demonstrate equivalent detection efficiency of all species investigated. The CT differences between individual tests were somewhat greater than those established for the Pan-AC detection assay. However, the data indicate that the differences between CT values were mainly attributable to inaccuracies in the measurement of fungal DNA concentrations.

### 1.2.4. Testing for cross-reactivity:

Both RQ-PCR assays were tested for possible cross-reactivity with bacterial or viral pathogens by sequence alignment using the BLAST software and experimentally by employing the Pan-AC and Pan-fungal assays for testing of DNA derived from a panel of more than 20 different bacterial and viral species (see Materials and Methods). No cross-reactivity between the Pan-AC and Pan-fungal primer-probe detection systems and bacterial or viral DNA investigated was observed.

As indicated in Fig. 1, human DNA sequences from different genome sites show high homology with the fungal DNA sequences targeted by the Pan-AC and Pan-fungal assays. Testing of human DNA extracted from peripheral blood of healthy volunteers by the Pan-AC assay revealed some degree of cross-reactivity. Different chemicals including dimethyl sulfoxide (DMSO), glycerol and formamide, which had been shown to increase the stringency of PCR reactions, were tested at concentrations ranging from 1% to 5% to assess the possibility of eliminating the cross-reactivity without compromising the overall amplification efficiency of the RQ-PCR assay. DMSO and glycerol completely inhibited the PCR reaction already at low concentrations. By contrast, formamide concentrations of 2% abrogated the cross-reactivity with human DNA, while maintaining a high amplification efficiency of the PCR assay. The Pan-fungal assay has not shown any cross-reactivity with human DNA specimens.

### 1.2.5. Sensitivity assessment of the assay:

The detection limit of the Pan-AC and Pan-fungal assay was determined by using primarily gDNA derived from A.fumigatus and C.albicans. The exact quantity of these two fungal species was calculated on the basis of fluorometric measurements, and serial logarithmic dilutions were prepared across a range of seven logs (1 fg-1 ng DNA). The logarithmic dilutions were tested in a series of experiments, as described below. Standard curves were generated on the basis of the amplification profile of the serial dilutions (see Figure 2 for the Pan-AC assay and Figure 3 for the Pan-fungal assay). The lower detection limit of the Pan-AC assay was 10 fg and of the Pan-fungal assay 1 fg fungal DNA per reaction, respectively. Nearly identical amplification efficiency and sensitivity of detection have been determined for other fungal species of interest. The standard curves established based on A. fumigatus and C.albicans dilutions were therefore applicable to quantitative analysis of other Aspergillus, Candida and other species.

The sensitivity of detection in clinical samples was determined by spiking fungal pathogens at defined concentrations into blood specimens. The achievable limit of detection was not impaired by the presence of a large excess of human DNA, permitting reproducible detection of one to five fungal genomes per milliliter PB for both assays.

### 1.2.6. Reproducibility of the assay:

Serial dilutions using purified DNA derived from A. fumigatus and C. albicans covering a seven log range (1 fg - 1 ng) were prepared. Moreover, peripheral blood samples spiked with A. fumigatus conidia and C. albicans cells, serially diluted across a range of 1E+0 to 1E+5 particles per milliliter, were subjected to the Pan-AC and Pan-fungal assays to assess the reproducibility of the techniques. The fungal DNA dilutions and the PB samples spiked with serially diluted fungi were tested in triplicates and all experiments were repeated twice. The CT values derived from the entire set of experimental data were used for the assessment of reproducibility by determining the intra-class coefficients of variation (ICC) Shrout et al., 1979). The reproducibility of the test system was shown to be excellent with regard to both inter-and infra-assay concordance, with ICC values >0.99 in all experiments performed.

### 1.3. Concluding remarks

In the present example, two RQ-PCR assays are described permitting the detection and quantification of a large number of Aspergillus and Candida species in a single PCR reaction, and of a broad range of clinically relevant molds and yeasts in a preferably two-reaction PCR assay. Hence, both RQ-PCR assays based on the TaqMan principle cover pathogens responsible for the vast majority of invasive fungal infections in immunosuppressed individuals in a single tube (Pan-AC) or dual tube (Panfugal) format. In comparison to earlier quantitative PCR approaches to the detection of fungal pathogens in the real-time TaqMan format (Costa et al., 2002) (Maaroufi et al., 2004) (Pham et al., 2003) (Sanguinetti et al., 2003), both assays presented provide considerably broader specificity, covering a large spectrum of clinically relevant Aspergillus and Candida species (Pan-AC assay) or a very broad range of important molds and yeasts (Pan-fungal assay) (Jordanides et al., 2005).

It has been suggested that in patients with IFI the fungal load in blood samples is generally low, frequently below 10 colony-forming units (c.f.u.) per milliliter PB (Loeffler et al., 2000). Based on this notion, the sensitivity of the PCR assay is crucial for the detection and reliable quantification of these pathogens. To achieve a limit of detection adequate for clinical screening, the assays presented were designed to target the fungal ribosomal RNA gene (rRNA), which is highly amplified in the fungal genome, with approximately 100 copies per organism (Maleszka et al., 1993). It has been shown that targeting of this multicopy gene by PCR permits the detection of 0.2 genome equivalents of S.cerevisiae (Sandhu et al., 1995).

To design molecular diagnostic tests covering the desired spectrum of Aspergillus and Candida species on one hand, and a broad range of molds and yeasts on the other hand, comprehensive sequence information on the fungi of interest was required. Detailed comparison of fungal DNA sequences by multiple alignment and serial BLAST searches has been performed to identify a target region within the rRNA gene revealing a high degree of homology across the fungal species and displaying a low level of similarity with DNA of non-fungal organisms. A number of possible target sequences has already been published for conventional pan-fungal PCR assays (Einsele et al., 1997(Hendolin et al., 2000) (Sandhu et al., 1995) (van Burik et al., 1998). However, the regions described earlier were not well-suited for the design of a RQ-PCR fungus detection system with broad specificity. For the Pan-AC and Pan-fungal RQ-PCR systems presented, a highly homologous region, displaying only very few nucleotide mismatches between the fungal species of interest, has been identified. For the Pan-AC assay two sequence-specific primers and a single TaqMan probe containing two wobble positions were designed and demonstrated to provide an efficient diagnostic tool for all Aspergillus and Candida species targeted. The Pan-fungal assay was designed as a two-reaction RQ-PCR test including detection reaction I, which targets predominantly filamentous fungi, and detection reaction II, which covers mainly yeasts and zygomycetes. In total, more than 50 clinically relevant fungal species were tested experimentally and were shown to be reliably covered by the Pan-fungal assay. Moreover, due to the high sequence homology of the region targeted by the assays, it can be expected that the systems will permit the detection of at least 30 additional species, as determined by sequence alignment analysis.

The assays were shown to lack cross-reactivity with bacterial or viral pathogens as well as with human DNA. By increasing the stringency of the RQ-PCR assay addition of 2% formamide to the reaction mixture further increased efficiency, without significantly affecting the fluorescence intensity and sensitivity of fungus detection. The Pan-fungal assay did not show any cross-reactivity with human DNA.

Contamination with extraneous fungus material is considered a major pitfall in PCR-based detection of fungal pathogens. The contamination with PCR products can be easily controlled by the combined use of dUTP and uracil N'-glycosylase treatment (see Materials and Methods section). A much greater and often underestimated problem is the potential contamination with air-borne fungal spores, which may occur during DNA extraction and PCR set-up. Perhaps even more importantly, both self-made and commercially available reagents may contain traces of fungal material. As yeasts and molds occur ubiquitously in the environment, also materials and reagents not produced under sterile conditions may be contaminated. It has been reported that Zymolase, an enzyme used in fungus DNA extraction, contained traces of DNA from S.cerevisiae (Loeffler et al., 1999(Rimek et al., 1999). Occasionally, batches of 10 x PCR buffer were found to be contaminated with DNA of Acremonium spp. (Loeffler et al., 1999). Multiple negative controls may be included in each assay to detect the occurrence of occasional contamination. If the fungus detection reaction is performed under suitable experimental conditions, including the preparation of reagents and processing of clinical samples under a laminar flow hood, and if all appropriate precautions and controls are considered, RQ-PCR can be regarded as a very powerful diagnostic tool for the detection of pathogenic fungi in clinical samples.

The Pan-AC and Pan-fungal assays presented are highly sensitive and permit quantitative analysis of fungal pathogens across a wide log range. Based on the economic design and low labor intensity, they can be readily used in clinical studies on fungus infection and the surveillance of the response to treatment. The assays can be completed within a few hours, starting from sample collection to the report of results, and can be easily implemented in any appropriately equipped clinical diagnostic laboratory.

### Example 2: Set-up of the Pan-fungus detection assay in a single reaction

The two-reaction Pan-fungus detection assay described in example 1 permits immediate distinction between Candida species and other pathogenic fungi on one hand and Aspergillus species and a number of other filamentous fungi on the other hand. In instances, in which the immediate separation into the two panels of fungi covered by the two-reaction assay is not required, the single-reaction Pan-fungus assay may be the detection method of choice offering the most economic approach to broad fungus screening.

The reduction to a single-reaction assay was made possible by identifying target sequences for a specifically modified probe permitting sensitive detection of the fungi covered by the two-reaction assay. Two alternative probes have been designed and tested including Pan-fungal probe I (sequence: ctt Gtk CGc tAt egg t) and Pan-fungal probe II (sequence: tac TtG tkC Gct Atc ggt), both carrying FAM as a 5'end modification and BHQ-1 as a 3'end modification. Capital letters in the sequence indicate LNA-modified nucleotides, the letter "k" identifies a wobble position for the nucleotides "g" or "t". Although both probes provided good results, Pan-fungal probe II is preferably used because said probe lead to superior results to those obtained with Pan-fungal probe I.

The single-reaction Pan-fungus assay is carried out in a total volume of 25 µl, and contains all primers included in the two-reaction assay (see Table 6, example 1) at the concentration indicated below.

There are different possibilities of primer combinations, which provide adequate amplification results. The main difference between the reaction set-up variants indicated are the amplicon lengths, which are shorter for Variant 1 by approximately 30-50nt, depending on the fungal species detected.

| Variant 1: | | |
|---|---|---|
| Master Mix | (Applied Biosystems) | 2x |
| Primers: | FI-A | 10 pmol |
| | FII-A | 10 pmol |
| | FIII-A | 10 pmol |
| | R-I | 10 pmol |
| | R-II | 10 pmol |
| Probe: | Pan-fungal II | 1,5 pmol |

| Variant 2: | | |
|---|---|---|
| Master Mix | (Applied Biosystems) | 2x |
| Primers: | FI-B | 10 pmol |
| | FII-B | 10 pmol |
| | FIII-B | 10 pmol |
| | R-I | 10 pmol |
| | R-II | 10 pmol |
| Probe: | Pan-fungal II 1,5 | pmol |

The amplification profile used is identical to the assays described above.

The single-reaction Pan-fungus assay has been tested for cross-reactivity with human DNA and was found to lack any non-specific signals in a series of experiments testing a panel of different human DNAs. The sensitivity of this assay was found to be equivalent to the two-reaction Pan-fungal assay.

### References:

Boutati, E. I., et al. (1997) Blood 90:999-1008.
Chen, S. C., et al. (2002) Med Mycol. 40:333-57.
Chen, Y. C., et al. (2000) J.Clin.Microbiol. 38:2302-2310.
Corpet, F. (1988) Nucleic Acids Res. 16:10881-10890.
Costa, C., et al. (2002) J.Clin.Microbiol. 40:2224-2227.
Costa, C., et al. (2001) J.Microbiol Methods 44:263-269.
De Baere, T., et al. (2002) BMC.Microbiol. 2:21.
Denning, D. W. (1998) Clin Infect. Dis. 26:781-803.
Einsele, H., et al. (1997) J.Clin.Microbiol. 35:1353-1360.
Ferrer, C., et al. (2001) J.Clin.Microbiol. 39:2873-2879.
Golan, Y. (2005) Am.J.Health Syst.Pharm. 62:S17-S21.
Hebart, H., et al. (1997) Onkologie 20:99-104.
Hendolin, P. H., et al. (2000) J.Clin.Microbiol..38:4186-4192.
Hopfer, R. L., et al. (1993) J.Med.Vet.Mycol. 31:65-75.
Hsu, M. C., et al. (2003) J.Med.Microbiol. 52:1071-1076.
Husain, S., et al. (2003) Clin.Infect.Dis. 37:221-229.
Ibanez-Nolla, J., et al. (2001) Mycoses 44:47-53.
Jaeger, E. E., et al. (2000) J.Clin.Microbiol. 38:2902-2908.
Jandrlic, M., et al. (1995) Eur.J.Clin.Microbiol.Infect.Dis. 14:768-774.
Jordanides, N. E., et al. (2005) Bone Marrow Transplant. 35:389-395.
Loeffler, J., et al. (1999) J.Clin.Microbiol. 37:1200-1202.
Loeffler, J., et al. (2000) J.Clin.Microbiol. 38:586-590.
Loffler, J., et al. (1997). J.Clin.Microbiol. 35:3311-3312. Maaroufi, Y., et al. (2004) J.Mol.Diagn. 6:108-114.
Machetti, M., et al. (1998) Bone Marrow Transplant. 21:917-921.
Maleszka, R., et al. (1993) Yeast 9:53-58.
Morace, G., et al. (1997) J.Clin.Microbiol. 35:667-672.
O'Brien, S. N., et al. (2003) In Hematology 2003, American Society of Hematology, Education Program Book. San Diego,
California, 438-472.
Pham, A. S., et al. (2003) Am.J.Clin.Pathol. 119:38-44.
Rath, P. M., et al. (2000) Mycoses 43:381-386.
Ribes, J. A., et al. (2000) Clin Microbiol Rev. 13:236-301.
Rimek, D., et al. (1999) J.Clin Microbiol. 37:830-831.
Sandhu, G. S., et al. (1995) J.Clin.Microbiol. 33:2913-2919.
Sanguinetti, M., et al. (2003) J.Clin.Microbiol. 41:3922-3925.
Shrout., P.E., et al. (1979) Psychological Bulletin 86:420-428.
Singh, N. (2001) Clin Infect.Dis. 33:1692-1696.
Turenne, C. Y., et al. (1999) J.Clin.Microbiol. 37:1846-1851.
van Burik, J. A., et al. (1998) J.Clin Microbiol. 36:1169-1175.
Vincent, J. L., et al. (1998) Intensive Care Med 24:206-216.
Watzinger, F., et al. (2004) J.Clin.Microbiol. 42:5189-5198.
White, P. L., et al. (2003) J.Med.Microbiol., 52:229-238.
Williamson, E. C., et al. (2000) Br.J.Haematol. 108:132-139.

### Sequence listing:

SEQ ID No. 1
SEQ ID No. 2
SEQ ID No. 3
SEQ ID No. 4
SEQ ID No. 5
SEQ ID No. 6
SEQ ID No. 7

## Claims

1. Method for the simultaneous detection of at least one fungus, in particular mold and yeast, in a sample comprising the steps of:
a) subjecting a sample suspected of containing at least one fungus to a detection reaction I by contacting the sample with forward primer 5'-TAAAGCTAAATAYYGGCCRGAGA-3' (SEQ ID No. 11) and reverse primers 5'-CTTTYCAAAGTGCTTTTCATC-3' (SEQ ID No. 19) and 5'-CTCTTTTCAAAGTWCTTTTCAYC-3' (SEQ ID No. 20) and a nucleic acid probe having the sequence 5'-ACTTGTGCGCTATCG-3' (SEQ ID No. 26) and to a detection reaction II by contacting the sample with forward primers 5'-GGGTGGTRARYTCCWTCTAARGCTAA-3' (SEQ ID No. 13) and 5'-GGGWGGTAAATCYCWCCTAAAGCTAA-3' (SEQ ID No. 14) and with reverse primers 5'-CTTTYCAAAGTGCTTTT-CATC-3' (SEQ ID No. 19) and 5'-CTCTTTTCAAAGTWCTTTTCAYC-3' (SEQ ID No. 20), and a nucleic acid probe having the sequence 5'-ACTTGTTCGCTATCG-3' (SEQ ID No. 35), wherein detection reaction I and detection reaction II is a nucleic acid amplification technique, and
b) optionally determining the presence of the at least one fungus in said sample by detecting a nucleic acid amplification product.

2. Method according to claim 1, **characterized in that** the at least one fungus is a member of a fungus genus selected from the group consisting of Aspergillus, Candida, Fusarium, Trichosporon, Mucor, Rhizopus, Acremonium, Cryptococcus, Scedosporium, Alternaria, Histoplasma, Penicillium, Bipolaris, Cladosporium, Maleassezia, Saccharomyces, Rhodotorula, Apophysomyces, Basidiobolus, Cokeromayces, Cunninghamella, Mortierella, Rhizomucor, Saksenaea, Geotrichum and combinations thereof.

3. Method according to claim 1 or 2, **characterized in that** the at least one fungus is selected from the group consisting of Aspergillus flavus, Aspergillus fumigatus, Aspergillus nidulans, Aspergillus niger, Aspergillus terreus, Aspergillus candidus, Aspergillus clavatue, Aspergillus ochraceus, Aspergillus penicillioides, Aspergillus ustus, Aspergillus versicolor, Candida albicans, Candida cylindracea, Candida dubliniensis, Candida famata, Candida glabrata, Candida guilliermondii, Candida inconspicua, Candida kefyr, Candida krusei, Candida lambica, Candida lusitaniae, Candida membranaefaciens, Candida norvegensis, Candida parapsilosis, Candida pelliculosa, Candida rugosa, Candida sake, Candida tropicalis, Candida utilis, Candida allociferii, Candida colliculosa, Candida lipolytica, Candida zeylanoides, Fusarium oxysporum, Fusarium proliferatum, Fusarium solani, Fusarium verticillioides, Acremonium strictum, Trichosporon asahii, Trichosporon cutaneum, Trichosporon inkin, Trichosporon moniliiforme, Trichosporon asteroides, Trichosporon ovoides, Trichosporon mucoides, Mucor circinelloides, Mucor hiemalis, Mucor mucedo, Mucor racemosus, Mucor ramosissimus, Mucor amphibiorum, Mucor rouxii, Rhizopus oryzae, Rhizopus azygosporus, Rhizopus microsporus, Rhizopus stolonifer, Absidia corymbifera, Cryptococcus albidus, Cryptococcus laurentii, Cryptococcus neoformans, Scedosporium apiospermum, Scedosporium proliferans, Alternaria alternata, Histoplasma capsulatum, Penicillium chrysogenum, Cladosporium cladosporioides, Cladosporium oxysporum, Blastoschizomyces capitatus, Malassezia furfur, Malassezia sympodialis, Saccharomyces cerevisiae, Rhodotorula mucilaginosa, Apophysomyces elegans, Basidiobolus ranarum, Cokeromyces recurvatus, Cunninghamella bertholletiae, Mortierella wolfii, Saksenaea vasiformis, Geotrichum candidum and combinations thereof.

4. Method according to claim 3, **characterized in that** the at least one nucleic acid probe is conjugated to a dye, preferably to a fluorescent dye, and optionally to a quencher.

5. Method according to any one of claims 1 to 4, **characterized in that** the nucleic acid amplification technique is a polymerase chain reaction technique.

6. Method according to claim 5, **characterized in that** the polymerase chain reaction technique is selected from the group consisting of real-time PCR, preferably TaqMan PCR, quantitative PCR, nested PCR, asymmetric PCR, multiplex PCR, inverse PCR, rapid PCR and combinations thereof.

7. Method according to any one of claims 1 to 6, **characterized in that** the amplified nucleic acid product is additionally tagged for detection by a technique selected from the group consisting of DNA-tagging by random-priming, DNA-tagging by nick-translation, DNA-tagging by polymerase chain reaction, oligonucleotide tailing, hybridization, tagging by kinase activity, fill-in reaction applying Klenow fragment, photobiotinylation and combinations thereof.

8. Method according to any one of claims 1 to 7, **characterized in that** the amplified nucleic acid product is detected by gel electrophoresis, Southern-blot, photometry, chromatography, colorimetry, fluorography, chemiluminescence, autoradiography, detection by specific antibody and combinations thereof.

9. Method according to any one of claims 1 to 8,' **characterized in that** the primers of the at least one forward and said at least one reverse primer or the at least one nucleic acid probe comprise at least one LNA (locked nucleic acid) nucleotide.

10. Method according to any one of claims 1 to 9, **characterized in that** a nucleic acid as positive control and a positive control specific primer pair and optionally a positive control specific nucleic acid probe derived from said nucleic acid is added to the sample after method step a), wherein said nucleic acid does not result in a nucleic acid amplification product when contacted and amplified with the at least one forward and the at least one reverse primer, and wherein the primer pair derived from said nucleic acid does not result in a nucleic acid amplification product when contacted with a sample free of any fungus.

11. Method according to claim 10, **characterized in that** the nucleic acid is human or phocine herpesvirus DNA.

12. Method according to claim 11, **characterized in that** the positive control specific primer pair comprises a forward primer 5'-TGAGTATGCCTGCCGTGTGA-3' (SEQ ID No. 27), a reverse primer 5'-ACTCATACACAACTTTCAGCAGCTTAC-3' (SEQ ID No. 28) and a positive control specific nucleic acid probe 5'-CCATGTGACTTTGTCACAGCCCAAGATAGTT-3' (SEQ ID No. 29), when human DNA is added to the sample, and a forward primer 5'-GGGCGAATCACAGATTGAATC-3' (SEQ ID No. 30), a reverse primer 5'--GCGGTTCCAAACGTACCAA-3' (SEQ ID No. 31) and optionally a positive control specific nucleic acid probe 5'-TTTTTATGTGTCCGCCACCATCTGGATC-3' (SEQ ID No. 32), when phocine herpesvirus DNA is added to the sample.

13. Use of a method according to any one of claims 1 to 12 for the detection of at least one fungus of the genus Aspergillus, Candida, Fusarium, Trichosporon, Mucor, Rhizopus, Acremonium, Cryptococcus, Scedosporium, Alternaria, Histoplasma, Penicillium, Bipolaris, Cladosporium, Maleassezia, Saccharomyces, Rhodotorula, Apophysomyces, Basidiobolus, Cokeromyces, Cunninghamella, Mortierella, Rhizomucor, Saksenaea and/or Geotrichum in a sample.

14. Kit for the simultaneous detection of at least one fungus in a sample comprising:
- forward primers 5'-TAAAGCTAAATAYYGGCCRGAGA-3' (SEQ ID No. 11), 5'-GGGTGGTAAAYTCCWTCTAARGCTAA-3' (SEQ ID No. 13) and 5'-GGGWGGTAAATCYCWCCTAAAGCTAA-3' (SEQ ID No. 14),
- reverse primers 5'-CTTTYCAAAGTGCTTTTCATC-3' (SEQ ID No. 19) and 5'-CTCTTTTCAAAGTWCTTTTCAYC-3' (SEQ ID No. 20),
- nucleic acid probes, comprising labelled nucleic acid probes 5'-ACTTGTGCGCTATCG-3' (SEQ ID No. 26) and 5'-ACTTGTTCGCTATCG-3' (SEQ ID No. 35),
- optionally a nucleic acid as positive control and a positive control specific primer pair and nucleic acid probe as defined in any one of claims 10 to 12.

## Patentansprüche

1. Verfahren zum gleichzeitigen Nachweis mindestens eines Pilzes, insbesondere Schimmel und Hefe, in einer Probe, umfassend die folgenden Schritte:
a) das Durchführen einer Nachweisreaktion I mit einer Probe, von der angenommen wird, dass sie mindestens einen Pilz enthält, durch das Inkontaktbringen der Probe mit dem Vorwärtsprimer 5'-TAAAGCTAAATAYYGGCCRGAGA-3' (SEQ ID NR: 11) und den Rückwärtsprimern 5'-CTTTYCAAAGTGCTTTTCATC-3' (SEQ ID NR: 19) und 5'-CTCTTTTCAAAGTWCTTTTCAYC-3' (SEQ ID NR: 20) und einer Nukleinsäuresonde, welche die Sequenz 5'-ACTTGTGCGCTATCG-3' (SEQ ID NR: 26) aufweist, sowie das Durchführen einer Nachweisreaktion II durch das Inkontaktbringen der Probe mit den Vorwärtsprimern 5'-GGGTGGTRARYTCCWTCTAARGCTAA-3' (SEQ ID NR: 13) und 5'-GGGWGGTAAATCYCWCCTAAAGCTAA-3' (SEQ ID NR: 14) und den Rückwärtsprimern 5'-CTTTYCAAAGTGCTTTTCATC-3' (SEQ ID NR: 19) und 5'-CTCTTTTCAAAGTWCTTTTCAYC-3' (SEQ ID NR: 20) und einer Nukleinsäuresonde, welche die Sequenz 5'-ACTTGTTCGCTATCG-3' (SEQ ID NR: 35) aufweist, wobei es sich bei der Nachweisreaktion I und der Nachweisreaktion II jeweils um eine Technik der Nukleinsäureamplifikation handelt; und
b) gegebenenfalls das Bestimmen des Vorliegens des mindestens einen Pilzes in der Probe anhand des Nachweises eines amplifizierten Nukleinsäureprodukts.

2. Verfahren nach Anspruch 1, **dadurch gekennzeichnet, dass** es sich bei dem mindestens einen Pilz um einen Vertreter einer Pilzgattung handelt, die ausgewählt ist aus der Gruppe bestehend aus Aspergillus, Candida, Fusarium, Trichosporon, Mucor, Rhizopus, Acremonium, Cryptococcus, Scedosporium, Alternaria, Histoplasma, Penicillium, Bipolaris, Cladosporium, Malassezia, Saccharomyces, Rhodotorula, Apophysomyces, Basidiobolus, Cokeromyces, Cunninghamella, Mortierella, Rhizomucor, Saksenaea, Geotrichum und Kombinationen aus diesen.

3. Verfahren nach Anspruch 1 oder 2, **dadurch gekennzeichnet, dass** der mindestens eine Pilz ausgewählt ist aus der Gruppe bestehend aus Aspergillus flavus, Aspergillus fumigatus, Aspergillus nidulans, Aspergillus niger, Aspergillus terreus, Aspergillus candidus, Aspergillus clavatus, Aspergillus ochraceus, Aspergillus penicillioides, Aspergillus ustus, Aspergillus versicolor, Candida albicans, Candida cylindracea, Candida dubliniensis, Candida famata, Candida glabrata, Candida guilliermondii, Candida inconspicua, Candida kefyr, Candida krusei, Candida lambica, Candida lusitaniae, Candida membranaefaciens, Candida norvegensis, Candida parapsilosis, Candida pelliculosa, Candida rugosa, Candida sake, Candida tropicalis, Candida utilis, Candida allociferii, Candida colliculosa, Candida lipolytica, Candida zeylanoides, Fusarium oxysporum, Fusarium proliferatum, Fusarium solani, Fusarium verticillioides, Acremonium strictum, Trichosporon asahii, Trichosporon cutaneum, Trichosporon inkin, Trichosporon moniliiforme, Trichosporon asteroides, Trichosporon ovoides, Trichosporon mucoides, Mucor circinelloides, Mucor hiemalis, Mucor mucedo, Mucor racemosus, Mucor ramosissimus, Mucor amphibiorum, Mucor rouxii, Rhizopus oryzae, Rhizopus azygosporus, Rhizopus microsporus, Rhizopus stolonifer, Absidia corymbifera, Cryptococcus albidus, Cryptococcus laurentii, Cryptococcus neoformans, Scedosporium apiospermum, Scedosporium proliferans, Alternaria alternata, Histoplasma capsulatum, Penicillium chrysogenum, Cladosporium cladosporioides, Cladosporium oxysporum, Blastoschizomyces capitatus, Malassezia furfur, Malassezia sympodialis, Saccharomyces cerevisiae, Rhodotorula mucilaginosa, Apophysomyces elegans, Basidiobolus ranarum, Cokeromyces recurvatus, Cunninghamella bertholletiae, Mortierella wolfii, Saksenaea vasiformis, Geotrichum candidum und Kombinationen aus diesen.

4. Verfahren nach Anspruch 3, **dadurch gekennzeichnet, dass** die mindestens eine Nukleinsäuresonde an einen Farbstoff, bevorzugt an einen Fluoreszenzfarbstoff, und gegebenenfalls an einen Quencher konjugiert ist.

5. Verfahren nach einem der Ansprüche 1 bis 4, **dadurch gekennzeichnet, dass** es sich bei der Technik der Nukleinsäureamplifikation um eine Technik der Polymerase-Kettenreaktion handelt.

6. Verfahren nach Anspruch 5, **dadurch gekennzeichnet, dass** die Technik der Polymerase-Kettenreaktion ausgewählt ist aus der Gruppe bestehend aus Echtzeit-PCR, bevorzugt TaqMan-PCR, quantitativer PCR, geschachtelter *(nested)* PCR, asymmetrischer PCR, Multiplex-PCR, inverser PCR, schneller PCR und Kombinationen aus diesen.

7. Verfahren nach einem der Ansprüche 1 bis 6, **dadurch gekennzeichnet, dass** das amplifizierte Nukleinsäureprodukt zusätzlich markiert ist, um durch eine Technik nachgewiesen werden zu können, die ausgewählt ist aus der Gruppe bestehend aus DNA-Markierung durch Zufallspriming, DNA-Markierung durch Nick-Translation, DNA-Markierung durch Polymerase-Kettenreaktion, Oligonukleotid-Tailing, Hybridisierung, Markierung durch Kinaseaktivität, Auffüllreaktion unter Verwendung eines Klenow-Fragments, Photobiotinylierung und Kombinationen aus diesen.

8. Verfahren nach einem der Ansprüche 1 bis 7, **dadurch gekennzeichnet, dass** der Nachweis des amplifizierten Nukleinsäureprodukts durch Gelelektrophorese, Southern-Blot, Photometrie, Chromatographie, Kolorimetrie, Fluorographie, Chemilumineszenz, Autoradiographie, Nachweis anhand von spezifischen Antikörpern und Kombinationen aus diesen erfolgt.

9. Verfahren nach einem der Ansprüche 1 bis 8, **dadurch gekennzeichnet, dass** die Primer des mindestens einen Vorwärtsprimers und des mindestens einen Rückwärtsprimers oder die mindestens eine Nukleinsäuresonde mindestens ein LNA *(locked nucleic acid*)-Nukleotid umfassen.

10. Verfahren nach einem der Ansprüche 1 bis 9, **dadurch gekennzeichnet, dass** eine Nukleinsäure als positive Kontrolle und ein für die positive Kontrolle spezifisches Primerpaar und gegebenenfalls eine für die positive Kontrolle spezifische Nukleinsäuresonde, die von der Nukleinsäure abgeleitet ist, im Anschluss an den Verfahrensschritt a) zu der Probe gegeben werden, wobei die Nukleinsäure nicht zum Erhalt eines amplifizierten Nukleinsäureprodukts führt, wenn diese mit dem mindestens einen Vorwärtsprimer und dem mindestens einen Rückwärtsprimer in Kontakt gebracht und amplifiziert wird, und wobei das Primerpaar, das von der Nukleinsäure abgeleitet ist, nicht zum Erhalt eines amplifizierten Nukleinsäureprodukts führt, wenn es mit einer Probe in Kontakt gebracht wird, die frei von jeglicher Art von Pilzen ist.

11. Verfahren nach Anspruch 10, **dadurch gekennzeichnet, dass** es sich bei der Nukleinsäure um Herpesvirus-DNA vom Menschen oder vom Seehund handelt.

12. Verfahren nach Anspruch 11, **dadurch gekennzeichnet, dass** das für die positive Kontrolle spezifische Primerpaar einen Vorwärtsprimer 5'-TGAGTATGCCTGCCGTGTGA-3' (SEQ ID NR: 27), einen Rückwärtsprimer 5'-ACTCATACACAACTTTCAGCAGCTTAC-3' (SEQ ID NR: 28) und eine für die positive Kontrolle spezifische Nukleinsäuresonde 5'-CCATGTGACTTTGTCACAGCCCAAGATAGTT-3' (SEQ ID NR: 29) umfasst, wenn DNA vom Menschen zu der Probe gegeben wird, und einen Vorwärtsprimer 5'-GGGCGAATCACAGATTGAATC-3' (SEQ ID NR: 30), einen Rückwärtsprimer 5'-GCGGTTCCAAACGTACCAA-3' (SEQ ID NR: 31) und gegebenenfalls eine für die positive Kontrolle spezifische Nukleinsäuresonde 5'-TTTTTATGTGTCCGCCACCATCTGGATC-3' (SEQ ID NR: 32) umfasst, wenn DNA vom Seehund zu der Probe gegeben wird.

13. Verwendung eines Verfahrens nach einem der Ansprüche 1 bis 12 zum Nachweis mindestens eines Pilzes der Gattung Aspergillus, Candida, Fusarium, Trichosporon, Mucor, Rhizopus, Acremonium, Cryptococcus, Scedosporium, Alternaria, Histoplasma, Penicillium, Bipolaris, Cladosporium, Malassezia, Saccharomyces, Rhodotorula, Apophysomyces, Basidiobolus, Cokeromyces, Cunninghamella, Mortierella, Rhizomucor, Saksenaea und/oder Geotrichum in einer Probe.

14. Kit zum gleichzeitigen Nachweis mindestens eines Pilzes in einer Probe, umfassend:
- die Vorwärtsprimer 5'-TAAAGCTAAATAYYGGCCRGAGA-3' (SEQ ID NR: 11), 5'-GGGTGGTRARYTCCWTCTAARGCTAA-3' (SEQ ID NR: 13) und 5'-GGGWGGTAAATCYCWCCTAAAGCTAA-3' (SEQ ID NR: 14);
- die Rückwärtsprimer 5'-CTTTYCAAAGTGCTTTTCATC-3' (SEQ ID NR: 19) und 5'-CTCTTTTCAAAGTWCTTTTCAYC-3' (SEQ ID NR: 20);
- Nukleinsäuresonden, umfassend die markierten Nukleinsäuresonden 5'-ACTTGTGCGCTATCG-3' (SEQ ID NR: 26) und 5'-ACTTGTTCGCTATCG-3' (SEQ ID NR: 35); und
- gegebenenfalls eine Nukleinsäure als positive Kontrolle und ein für die positive Kontrolle spezifisches Primerpaar sowie eine wie in einem der Ansprüche 10 bis 12 definierte Nukleinsäuresonde.

## Revendications

1. Procédé pour la détection simultanée d'au moins un champignon, en particulier une moisissure et une levure, dans un échantillon, comprenant les étapes consistant à :
a) soumettre un échantillon suspecté de contenir au moins un champignon à une réaction de détection I en mettant en contact l'échantillon avec l'amorce sens 5'-TAAAGCTAAATAYYGGCCRGAGA-3' (SEQ ID NO: 11) et les amorces antisens 5'-CTTTYCAAAGTGCTTTTCATC-3' (SEQ ID NO: 19) et 5'-CTCTTTTCAAAGTWCTTTTCAYC-3' (SEQ ID NO: 20) et une sonde d'acide nucléique ayant la séquence 5'-ACTTGTGCGCTATCG-3' (SEQ ID NO: 26), et à une réaction de détection II en mettant en contact l'échantillon avec les amorces sens 5'-GGGTGGTRARYTCCWTCTAARGCTAA-3' (SEQ ID NO: 13) et 5'-GGGWGGTAAATCYCWCCTAAAGCTAA-3' (SEQ ID NO: 14) et avec les amorces antisens 5'-CTTTYCAAAGTGCTTTT-CATC-3' (SEQ ID NO: 19) et 5'-CTCTTTTCAAAGTWCTTTTCAYC-3' (SEQ ID NO: 20), et une sonde d'acide nucléique ayant la séquence 5'-ACTTGTTCGCTATCG-3' (SEQ ID NO: 35), où la réaction de détection I et la réaction de détection II est une technique d'amplification d'acide nucléique, et
b) déterminer facultativement la présence du au moins un champignon dans ledit échantillon en détectant un produit d'amplification d'acide nucléique.

2. Procédé selon la revendication 1, **caractérisé en ce que** le au moins un champignon est un membre d'un genre de champignon sélectionné dans le groupe consistant en Aspergillus, Candida, Fusarium, Trichosporon, Mucor, Rhizopus, Acremonium, Cryptococcus, Scedosporium, Alternaria,
Histoplasma, Penicillium, Bipolaris, Cladosporium, Malassezia, Saccharomyces, Rhodotorula, Apophysomyces, Basidiobolus, Cokeromyces, Cunninghamella, Mortierella, Rhizomucor, Saksenaea, Geotrichum et des combinaisons de ceux-ci.

3. Procédé selon la revendication 1 ou 2, **caractérisé en ce que** le au moins un champignon est sélectionné dans le groupe consistant en Aspergillus flavus, Aspergillus fumigatus, Aspergillus nidulans, Aspergillus niger, Aspergillus terreus, Aspergillus candidus, Aspergillus clavatus, Aspergillus ochraceus, Aspergillus penicillioides, Aspergillus ustus, Aspergillus versicolor, Candida albicans, Candida cylindracea, Candida dubliniensis, Candida famata, Candida glabrata, Candida guilliermondii, Candida inconspicua, Candida kefyr, Candida krusei, Candida lambica, Candida lusitaniae, Candida membranaefaciens, Candida norvegensis, Candida parapsilosis, Candida pelliculosa, Candida rugosa, Candida sake, Candida tropicalis, Candida utilis, Candida allociferii, Candida colliculosa, Candida lipolytica, Candida zeylanoides, Fusarium oxysporum, Fusarium proliferatum, Fusarium solani, Fusarium verticillioides, Acremonium strictum, Trichosporon asahii, Trichosporon cutaneum, Trichosporon inkin, Trichosporon moniliforme, Trichosporon asteroides, Trichosporon ovoides, Trichosporon mucoides, Mucor circinelloides, Mucor hiemalis, Mucor mucedo, Mucor racemosus, Mucor ramosissimus, Mucor amphibiorum, Mucor rouxii, Rhizopus oryzae, Rhizopus azygosporus, Rhizopus microsporus, Rhizopus stolonifer, Absidia corymbifera, Cryptococcus albidus, Cryptococcus laurentii, Cryptococcus neoformans, Scedosporium apiospermum, Scedosporium proliferans, Altemaria altemata, Histoplasma capsulatum, Penicillium chrysogenum, Cladosporium cladosporioides, Cladosporium oxysporum, Blastoschizomyces capitatus, Malassezia fusfur, Malassezia sympodialis, Saccharomyces cerevisiae, Rhodotorula mucilaginosa, Apophysomyces elegans, Basidiobolus ranarum, Cokeromyces recurvatus, Cunninghamella bertholletiae, Mortierella wolfii, Saksenaea vasiformis, Geotrichum candidum et des combinaisons de ceux-ci.

4. Procédé selon la revendication 3, **caractérisé en ce que** la au moins une sonde d'acide nucléique est conjuguée à un colorant, de préférence à un colorant fluorescent, et facultativement à un désactivateur.

5. Procédé selon l'une quelconque des revendications 1 à 4, **caractérisé en ce que** la technique d'amplification d'acide nucléique est une technique de réaction en chaîne par polymérase.

6. Procédé selon la revendication 5, **caractérisé en ce que** la technique de réaction en chaîne par polymérase est sélectionnée dans le groupe consistant en une PCR en temps réel, de préférence une PCR TaqMan, une PCR quantitative, une PCR nichée, une PCR asymétrique, une PCR multiplexe, une PCR inverse, une PCR rapide et des combinaisons de celles-ci.

7. Procédé selon l'une quelconque des revendications 1 à 6, **caractérisé en ce que** le produit d'acide nucléique amplifié est en outre marqué pour la détection par une technique sélectionnée dans le groupe consistant en un marquage de l'ADN par amorçage aléatoire, un marquage de l'ADN par translation de coupure, un marquage de l'ADN par une réaction en chaîne par polymérase, une addition aux extrémités d'un oligonucléotide, une hybridation, un marquage par une activité kinase, une réaction de comblement en appliquant un fragment Klenow, une photobiotinylation et des combinaisons de ceux-ci.

8. Procédé selon l'une quelconque des revendications 1 à 7, **caractérisé en ce que** le produit d'acide nucléique amplifié est détecté par électrophorèse sur gel, transfert de Southern, photométrie, chromatographie, colorimétrie, fluorographie, chimiluminescence, autoradiographie, détection par un anticorps spécifique et des combinaisons de ceux-ci.

9. Procédé selon l'une quelconque des revendications 1 à 8, **caractérisé en ce que** les amorces de la au moins une amorce sens et ladite au moins une amorce antisens ou la au moins une sonde d'acide nucléique comprennent au moins un nucléotide d'un LNA (acide nucléique bloqué).

10. Procédé selon l'une quelconque des revendications 1 à 9, **caractérisé en ce qu'**un acide nucléique en tant que contrôle positif et une paire d'amorces spécifique au contrôle positif, et facultativement une sonde d'acide nucléique spécifique au contrôle positif dérivée dudit acide nucléique, sont ajoutés à l'échantillon après l'étape a) du procédé, où ledit acide nucléique ne génère aucun produit d'amplification d'acide nucléique lorsqu'il est mis en contact et amplifié avec la au moins une amorce sens et la au moins une amorce antisens, et où la paire d'amorces dérivée dudit acide nucléique ne génère aucun produit d'amplification d'acide nucléique lorsqu'elle est mise en contact avec un échantillon qui ne contient aucun champignon.

11. Procédé selon la revendication 10, **caractérisé en ce que** l'acide nucléique est un ADN humain ou d'herpèsvirus de phoque.

12. Procédé selon la revendication 11, **caractérisé en ce que** la paire d'amorces spécifique au contrôle positif comprend une amorce sens 5'-TGAGTATGCCTGCCGTGTGA-3' (SEQ ID NO: 27), une amorce antisens 5'-ACTCATACACAACTTTCAGCAGCTTAC-3' (SEQ ID NO: 28) et une sonde d'acide nucléique spécifique au contrôle positif 5'-CCATGTGACTTTGTCACAGCCCAAGATAGTT-3' (SEQ ID NO: 29), lorsqu'un ADN humain est ajouté à l'échantillon, et une amorce sens 5'-GGGCGAATCACAGATTGAATC-3' (SEQ ID NO: 30), une amorce antisens 5'-GCGGTTCCAAACGTACCAA-3' (SEQ ID NO: 31) et facultativement une sonde d'acide nucléique spécifique au contrôle positif 5'-TTTTTATGTGTCCGCCACCATCTGGATC-3' (SEQ ID NO: 32), lorsqu'un ADN d'herpèsvirus de phoque est ajouté à l'échantillon.

13. Utilisation d'un procédé selon l'une quelconque des revendications 1 à 12, pour la détection d'au moins un champignon du genre Aspergillus, Candida, Fusarium, Trichosporon, Mucor, Rhizopus, Acremonium, Cryptococcus, Scedosporium, Alternaria, Histoplasma, Penicillium, Bipolaris, Cladosporium, Malassezia, Saccharomyces, Rhodotorula, Apophysomyces, Basidiobolus, Cokeromyces, Cunninghamella, Mortierella, Rhizomucor, Saksenaea et/ou Geotrichum dans un échantillon.

14. Kit pour la détection simultanée d'au moins un champignon dans un échantillon, comprenant :
- les amorces sens 5'-TAAAGCTAAATAYYGGCCRGAGA-3' (SEQ ID NO: 11), 5'-GGGTGGTRARYTCCWTCTAARGCTAA-3' (SEQ ID NO: 13) et 5'-GGGWGGTAAATCYCWCCTAAAGCTAA-3' (SEQ ID NO: 14),
- les amorces antisens 5'-CTTTYCAAAGTGCTTTTCATC-3' (SEQ ID NO: 19) et 5'-CTCTTTTCAAAGTWCTTTTCAYC-3' (SEQ ID NO: 20),
- des sondes d'acide nucléique, comprenant les sondes d'acide nucléique marquées 5'-ACTTGTGCGCTATCG-3' (SEQ ID NO: 26) et 5'-ACTTGTTCGCTATCG-3' (SEQ ID NO: 35),
- facultativement un acide nucléique en tant que contrôle positif et une paire d'amorces et une sonde d'acide nucléique spécifiques au contrôle positif tel que défini dans l'une quelconque des revendications 10 à 12.
